# EUROPEAN PATENT APPLICATION

(11) **EP 2 441 758 A1**
(43) Date of publication of application: **18.04.2012**
(21) Application number: 10785786.4
(22) Date of filing: 11.06.2010
(51) Int. Cl.: C07D 261/20, C07D 307/20, C07D 491/22, C07D 205/12, C07F 7/08

(54) **SYNTHESIS OF TTX INTERMEDIATES**

(30) Priority: 12.06.2009 ES 200901409
(71) Applicant: Consejo Superior De Investigaciones Científicas, 28006 Madrid (ES)
(72) Inventor: NOHEDA MARÍN, Pedro, E-28006 Madrid (ES); LOZANO GORDILLO, Luis Miguel, E-28006 Madrid (ES); TABARES CANTERO, Nuria, E-28006 Madrid (ES); BENITO ARENAS, Raúl, E-28006 Madrid (ES); HERRERO RUIZ, David, E-28049 Madrid (ES)
(74) Representative: ABG Patentes, S.L.
(86) International application number: PCT/ES2010/070393
(87) International publication number: WO 2010/142836

(57) **Abstract**

The present invention relates to the synthesis of intermediates which are useful in TTX synthesis and to the preparation thereof.

## Description

### Field of the Invention

The present invention relates to the synthesis of TTX intermediates and their analogs.

### Background of the Invention

Tetrodotoxin (TTX), octahydro-12-(hydroxymethyl)-2-imino-5,9:7,10a-dimethano-10aH-[1,3]dioxocino[6,5-d]pyrimidine-4,7,10,11,12-pentol is a powerful neurotoxin which selectively binds to the sodium channel proteins inhibiting their cell membrane function. Tetrodotoxin was isolated for the first time in 1909 from the ovaries of puffer fish and was named after the puffer fish "tetraodontidae" family. Despite being a small molecule, it has an extremely complex structure characterized by a dioxaadamantane skeleton, a guanidine residue which is part of a hemiaminal and an orthoacid bridge.

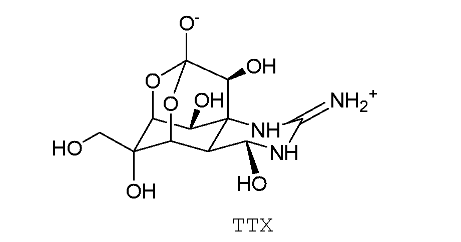

It must also be highlighted that TTX is normally present as a mixture of two possible tautomers: an orthoester and a hydroxylactone (Scheme 1). The ratio of both tautomers depends on the medium in which the TTX is present.

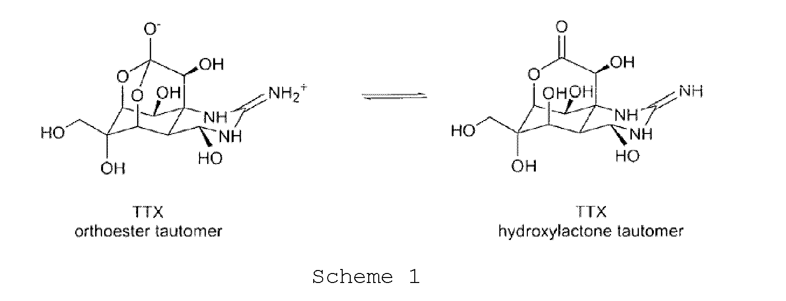

Due to its novel structure and high density of functional groups, only after extensive efforts did Hirata-Goto *et al.* (Goto, T.; Kishi, Y.; Takahashi, S.; Hirata, Y., Tetrahedron 1965, 21, 2059-2088), Tsuda *et al.* (Tsuda, K.; Ikuma, S.; Kawamura, M.; Tachikawa, K.; Sakai, K.; Tamura, C.; Amakasu, O., Chem. Pharm. Bull. 1964, 12, 1357-1374) and Woodward *et al.* (Woodward, R. B., Pure Appl. Chem. 1964, 9, 49-74; Woodward, R. B.; Gougoutas, J. Z., J. Am. Chem. Soc. 1964, 86, 5030) independently arrived at the same structure in 1964. Finally, in 1970 absolute stereochemistry was determined unambiguously by X-ray crystallographic analysis of its derivative (Furusaki, A.; Tomie, Y.; Nitta, I. Bull. Chem. Soc. Jpn. 1970, 43, 3325-3331).

Tetrodotoxin blocks sodium diffusion through the sodium channel, preventing depolarization and propagation of action potentials in nerve cells. The TTX-Na channel binding site is extremely tight (Kd = 10⁻¹⁰ nM). Therefore it is an essential tool in pharmacological studies related to sodium channel proteins.

Furthermore, tetrodotoxin, due to its analgesic properties, is a promising new drug candidate in the field of pain management.

Extraction and purification of natural TTX is complicated and depends on the availability of the suitable animal source. There is therefore an existing need of providing larger amounts of tetrodotoxin and its analogues, and researchers seek new inexpensive and efficient synthesis. Up until now, only a handful of total syntheses have been described (Kishi, Y.; Nakatsubo, F.; Aratani, M.; Goto, T.; Inoue, S.; Kakoi, H.; Tetrahedron Lett. 1970, 59, 5127-5128; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9217-9219; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9219-9220; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9220-9221), Isobe *et al.* in January 2003 (Isobe,M. et al, J. Am. Chem. Soc, 2003, 125, 8798-8805 ). Dubois *et al.* in June 2003 (Du Bois, J.; Hinman, A. J. Am. Chem. Soc. 2003, 125, 11510-11511), and Isobe *et al*. have described an additional asymmetric total synthesis comprising 62 steps and an overall yield of approximately 1% from a vinilic methyl intermediate (Isobe, M.; Urabe, D.; Nishikawa, T. Angew. Chem. Int. Ed. 2004, 43, 4782-4785). Further improvements by the same author have been published in 2006 (Isobe, M.; Urabe, D.; Nishikawa, T.; Urabe, D. Chem. Asian. J. 2006, 1-2, 125-135).

For the purposes of the present invention "TTX analogs" are those described in US 5,846,975 (included herein by reference). From column 3 line 40 to column 6 line 40 US 5,846,975 describes a general formula of known TTX analogs, for example, anhydroustetrodotoxin, tetrodaminotoxin, methoxytetrodotoxin, ethoxytetrodotoxin, deoxytetrodotoxin and tetrodonic acid, 6-epi-tetrodotoxin, 11-deoxytetrodotoxin as well as hemilactal type TTX derivatives (e.g. 4-*epi*-TTX, 6-*epi*-TTX, 11-*deoxy*-TTX, 4-*epi*-11-*deoxy*-TTX, TTX-8-O-hemisuccinate, chiriquitoxin, 11-*nor*-TTX-6(S)-ol, 11-*nor*-TTX-6(R)-ol, 11-*nor*-TTX-6,6-diol, 11-*oxo*-TTX and TTX-11-carboxylic acid), lactone type TTX derivatives (e.g. 6-*epi*-TTX (lactone), 11-*deoxy*-TTX (lactone), 11-*nor*-TTX-6(S)-ol (lactone), 11-*nor*-TTX-6(R)-ol (lactone), 11-*nor*-TTX-6,6-diol (lactone), 5-*deoxy*-TTX, 5,11-*dideoxy*-TTX, 4-*epi*-5,11-*dideoxy*-TTX, 1-*hydroxy*-5,11-*dideoxy*-TTX, 5,6,11-*trideoxy*-TTX and 4-*epi*-5,6,11-*trideoxy*-TTX) and 4,9-anhydrous type TTX analogs (e.g. 4,9-*anhydrous*-TTX, 4,9-*anhydrous*-6-*epi-*TTX, 4,9-*anhydrous*-11-*deoxy*-TTX, 4,9-*anhydrous*-TTX-8-O-hemisuccinate, 4,9-*anhydrous*-TTX-11-O-hemisuccinate). The typical TTX analogs possess only 1/8 to 1/40 of the TTX toxicity in mice, based upon bioassay in mice. It has been observed that these derivatives produce joint action and do not interact adversely.

Isobe also described the synthesis of different non-natural tetrodotoxin analogs following similar strategies as those described for tetrodotoxin. Specifically, Isobe *et al*. described the synthesis of (-)-5,11-dideoxytetrodotoxin (Isobe, M.; Asai, M.; Ohyabu, N.; Yamamoto, N.; Nishikawa, T. Angew. Chem. Int. Ed. 1999, 38, 3081-3084), (-)-8,11-dideoxytetrodotoxin (Isobe, M.; Asai, M.; Iwabuchi, T.; Yoshida, K.; Urabe, D.; Nishikawa, T. Org. Lett. 2002, 16, 2679-2682; Isobe, M.; Asai, M.; Iwabuchi, T.; Yoshida, K.; Urabe, D.; Nishikawa, T. Chem. Eur. J. 2004, 10, 452-462) and 11-deoxytetrodotoxin (Isobe, M.; Asai, Nishikawa, T. J. Am. Chem. Soc. 2002, 124, 7847-7852).

Sato *et al*. described a racemic total synthesis of tetrodotoxin from myo-inositol with approximately 0.14% overall yield after 40 steps (Sato, K.; Akai, S.; Sugita, N.; Ohsawa, T.; Kogure, T.; Shoji, H.; Yoshimura, J. J. Org. Chem. 2005, 70, 7496-7504 ).

Finally, Sato *et al*. recently described an asymmetric total synthesis of tetrodotoxin from D-glucose with approximately 0.13% overall yield after 40 steps (Sato, K.; Akai, S.; Sugita, N.; Yoshimura, J. J. Org. Chem. 2008, 73, 1234-1242).

Furthermore, additional discussions relating to different synthetic methods for tetrodotoxin and its analogs can also be reviewed in Koert, U. T. Angew. Chem. Int. Ed. 2004, 43, 5572-55769.

International application PCT/EP2008/051653 describes 1-azaspiro[3.5]nonan-2-one-5,7-carbolactone and 5,7-protected-1-azaspiro[3.5]nonan-2-one derivatives.

Hydroxylated lactams serving as starting material for the derivatives of the present invention are prepared in international application PCT/EP2005/005149.

In view of all of the above there is an existing need of providing an alternative inexpensive and efficient synthesis of tetrodotoxin and analogs thereof, which can be used for the industrial production of sufficient amounts of these compounds. Furthermore, new analogues with useful biological and pharmacological properties will be readily available by means of new synthetic strategies.

### Summary of the Invention

The authors of the invention, on their continuous effort to discover an industrially viable synthesis, have discovered that the intermediates of the invention are promising intermediate products for synthesizing TTX and analogs thereof. Therefore, a first aspect of the invention relates to a compound of formula (II), stereoisomers, salts or solvates thereof,

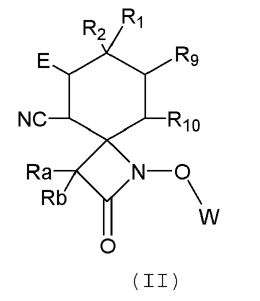

The preparation of the compounds of formula (II) allows efficiently functioning positions 4a and 5 of TTX according to the TTX numbering while at the same time introduces carbon 4. Additionally, it opens the door to the synthesis of more advanced intermediates in the TTX synthesis, such as the compounds of formula (III), (IV), (V), (VI), (VII), (VIII), (IX), (Xa), (XI), (XII), (XIII) and (XIV) each of which forms an additional aspect of the invention.

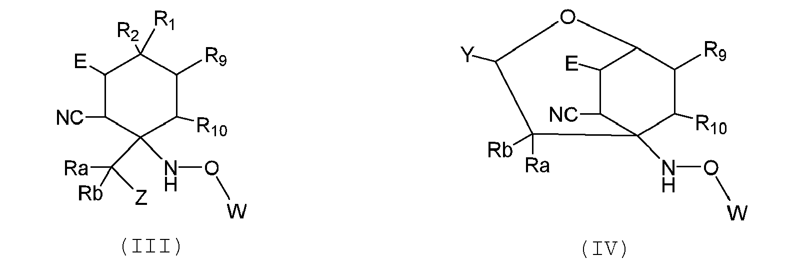

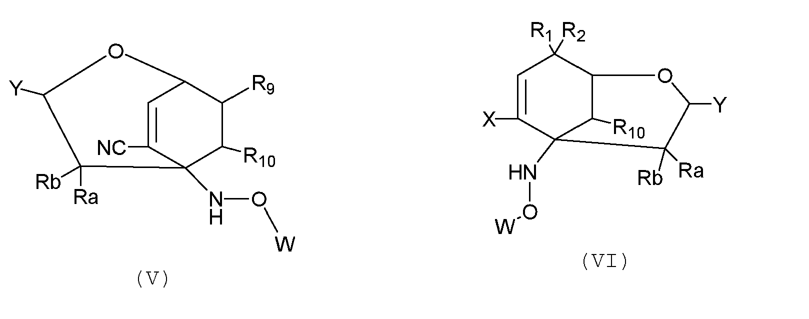

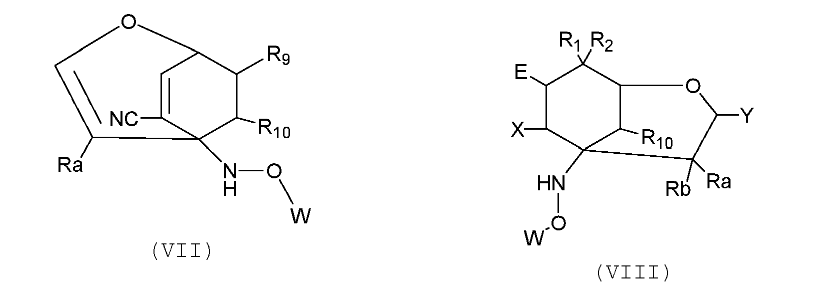

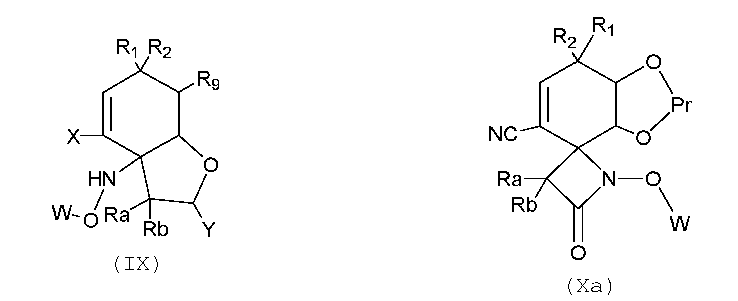

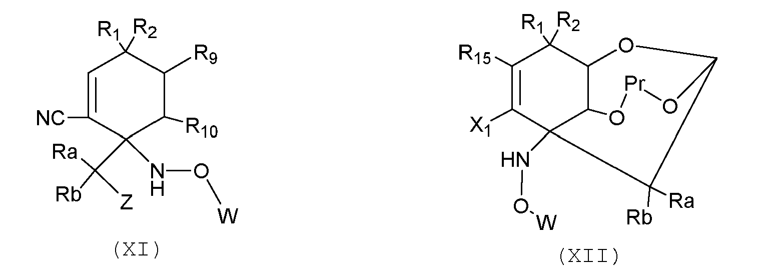

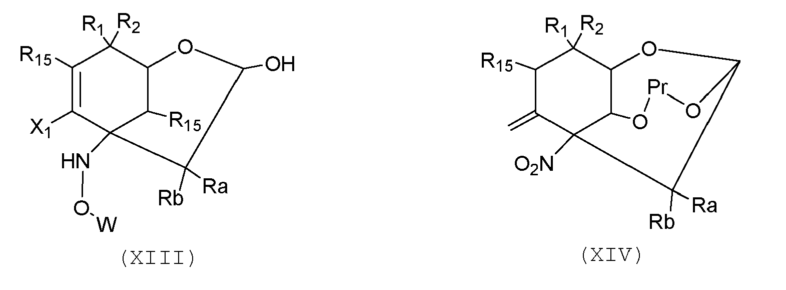

Additional aspects of the invention are methods for synthesizing said compounds of formula (II), (III), (IV), (V), (VI), (VII), (VIII), (IX), (IX), (Xa), (XI), (XII), (XIII) and (XIV) as well as their use as intermediates in the synthesis of TTX and analogs thereof. These compounds, as well as their more particular embodiments are called "compounds of the invention" throughout the present invention.

These intermediates of the invention form advanced intermediates in the TTX synthesis. Additionally, the synthesis also allows obtaining TTX analogs by means of modifying the different positions in a flexible manner. Methods similar to those described in other documents mentioned above (for example, in the Sato or Isobe publications) allow constructing the orthoester and the guanidinium ring from the compounds of the invention.

### Detailed Description of the Invention

### Definitions

Except in the examples, the numbering followed to designate the positions in the compounds of the invention has been the following:

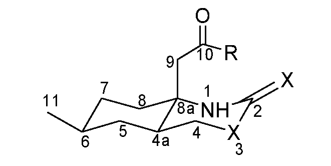

In other words, this is the numbering referred to when it is indicated that the number of a specific position is "according to the TTX numbering".

In the present document the following terms have the indicated meanings:
"Alkyl" refers to a straight or branched hydrocarbon chain radical consisting of carbon and hydrogen atoms, containing no saturation, having 1-12, preferably one to eight, more preferably one to six carbon atoms, and which is bound to the rest of the molecule by a single bond, e.g., methyl, ethyl, n-propyl, i-propyl, n-butyl, t-butyl, n-pentyl, etc.
"Alkenyl" refers to a straight or branched hydrocarbon chain radical consisting of carbon and hydrogen atoms, containing at least one unsaturation, having 2-12, preferably two to eight, preferably two to six carbon atoms, and which is bound to the rest of the molecule by a single bond.
"Alkylidene" refers to straight or branched hydrocarbon chain radical consisting of carbon and hydrogen atoms, having 1-12, preferably one to eight, more preferably one to six carbon atoms, and which is bound to the rest of the molecule by a double bond, e.g., methylene (=CH₂) or ethylidene (=CHCH₃).
"Alkynyl" refers to a straight or branched hydrocarbon chain radical consisting of carbon and hydrogen atoms, containing at least one carbon-carbon triple bond, conjugated or not, having two to twelve, preferably two to eight, preferably two to six carbon atoms, and which is bound to the rest of the molecule by a single bond, such as -CCH, -CH₂CCH, -CCCH₃, - CH₂CCCH₃.
"Aryl" or "Ar" refers to an aromatic hydrocarbon radical such as phenyl, naphthyl or anthracyl.
"Arylalkyl" refers to an alkyl group bound to the rest of the molecule through an alkyl group such as benzyl and phenethyl.
"Cycloalkyl" refers to cyclic hydrocarbon chain radical consisting of carbon and hydrogen atoms, containing no saturation, having 3 to 8 carbon atoms, bound to the rest of the molecule by a single bond.
"Alkoxyl" refers to a radical of the formula -Oalkyl, e.g., methoxy, ethoxy, propoxy, etc.
"Aryloxy" refers to a radical of formula -Oaryl.
"Heterocyclyl" refers to a stable 3- to 15-membered ring consisting of carbon atoms and one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle can be a monocyclic, bicyclic or tricyclic ring system, which can include fused ring systems; and the nitrogen, carbon or sulfur atoms in the heterocyclyl radical can be optionally oxidized; the nitrogen atom can be optionally quaternized; and the heterocyclyl radical can be partially or completely saturated or aromatic. Examples of said heterocycles include, but are not limited to, azepines, benzimidazole, benzothiazole, furan, isothiazole, imidazole, indole, piperidine, piperazine, purine, quinoline, thiadiazole, tetrahydrofuran.
"Amino" refers to a radical of formula -NH2, -NHR", - NR"R"', wherein R" and R"' independently represent a group selected from the group consisting of substituted or unsubstituted alkyl, substituted or unsubstituted alkenyl, substituted or unsubstituted alkynyl, substituted or unsubstituted aryl and substituted or unsubstituted arylalkyl.
"Hydroxyl protecting group" refers to a group blocking the OH function for subsequent reactions and can be removed under controlled conditions. Hydroxyl protecting groups are well known in the art, representative protecting groups are:
   - silyl ethers of formula -Si(R')3, such as trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether;
   - alkyl and arylalkyl ethers such as methyl ether, tert-butyl ether, benzyl ether, p-methoxybenzyl ether , 3,4-dimethoxybenzyl ether, trityl ether; allyl ether;
   - alkoxymethyl and aryloxy ethers of formula -CH₂-O-R', such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers; methylthiomethyl ether;
   - esters of formula -C(=O)R' such as acetate ester, benzoate ester; pivalate ester, methoxyacetate ester, chloroacetate ester, levulinate ester;
   - carbonates of formula -C(=O)-O-R' such as benzyl carbonate, p-nitrobenzyl carbonate, tert-butyl carbonate, 2,2,2-trichloroethyl carbonate, 2-(trimethylsilyl)ethyl carbonate, allyl carbonate; and
   - sulphates such as SO₃.py.

In all the above formula R' represents a group selected from the group consisting of substituted or unsubstituted alkyl, substituted or unsubstituted alkenyl, substituted or unsubstituted alkynyl, substituted or unsubstituted aryl and substituted or unsubstituted arylalkyl. Additional examples of hydroxyl protecting groups can be found in reference books such as Greene and Wuts "Protective Groups in Organic Synthesis", John Wiley & Sons, Inc., New Jersey, 2007.

"Amino protecting group" refers to a group blocking the NH₂ function for subsequent reactions and can be removed under controlled conditions. Amino protecting groups are well known in the art, representative protecting groups are carbamates, e.g. carbamates of formula -C(=O)OR'; amides, e.g. amides of formula -C(=O)R', such as substituted or unsubstituted acetates; or silyl moieties of formula -Si(R')3; wherein R' is as defined above. Different alkyl moeties can also serve as amino protecting groups. Said alkyl groups can optionally be substituted with one or more substituents such as halogen, hydroxyl, alkoxyl, alkyloxymethyl ethers, carboxy, cyano, carbonyl, acyl, alkoxycarbonyl, amino, nitro, mercapto and alkylthio. Additional examples of amino protecting groups can be found in reference books such as Greene and Wuts "Protective Groups in Organic Synthesis", John Wiley & Sons, Inc., New Jersey, 2007.

References herein to substituted groups in the compounds of the present invention refer to the specified moiety that may be substituted at one or more available positions with one or more suitable groups, e.g., halogen such as fluorine, chlorine, bromine and iodine; cyano; hydroxyl; nitro; azido; alkanoyl such as a C₁₋₆ alkanoyl such as acyl and the like; carboxamido; alkyl groups including those groups having 1 to 12 carbon atoms or 1 to 6 carbon atoms and more preferably 1-3 carbon atoms; alkenyl and alkynyl groups including groups having one or more unsaturated bonds and 2 to 12 carbons or 2 to 6 carbon atoms; alkoxyl groups having one or more oxygen bonds and 1 to 12 carbon atoms or 1 to approximately 6 carbon atoms; alkoxyl such as phenoxy; alkylthio groups including those moieties having one or more thioether bonds and 1 to about 12 carbon atoms or 1 to 6 carbon atoms; alkylsulfinyl groups including those moieties having one or more sulfinyl bonds and 1 to 12 carbon atoms or 1 to 6 carbon atoms; alkylsulfonyl groups including those moieties having one or more sulfonyl bonds and 1 to 12 carbon atoms or 1 to 6 carbon atoms; aminoalkyl groups such as those groups having one or more N atoms and 1 to 12 carbon atoms or 1 to 6 carbon atoms; carbocylic aryl having 6 or more carbons, particularly phenyl or naphthyl and arylalkyl such as benzyl. Unless otherwise indicated, an optionally substituted group can have a substituent at each substitutable position of the group, and each substitution is independent of the other.

Unless otherwise stated, it is understood that the compounds of the invention also include compounds which differ only in the presence of one or more isotopically enriched atoms. For example, compounds of formula (II), (III), (IV), (V), (VI), (VII), (VIII), (IX), (Xa), (XI), (XII), (XIII) or (XIV) wherein a hydrogen has been substituted with a deuterium or tritium, and/or the substitution of a carbon with ¹³C- or ¹⁴C-enriched carbon, and/or ¹⁵N-enriched nitrogen and/or the substitution of an oxygen with ¹⁸O, are within the scope of this invention.

### Compounds of the Invention

As indicated above, aspects of the present invention are the compounds of formula (II), (III), (IV), (V), (VI), (VII), (VIII), (IX), (Xa), (XI), (XII), (XIII) or (XIV), stereoisomers, salts or solvates thereof, 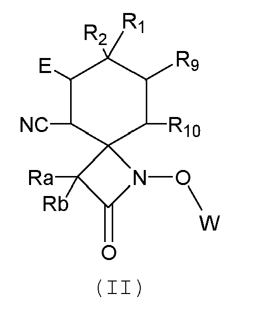 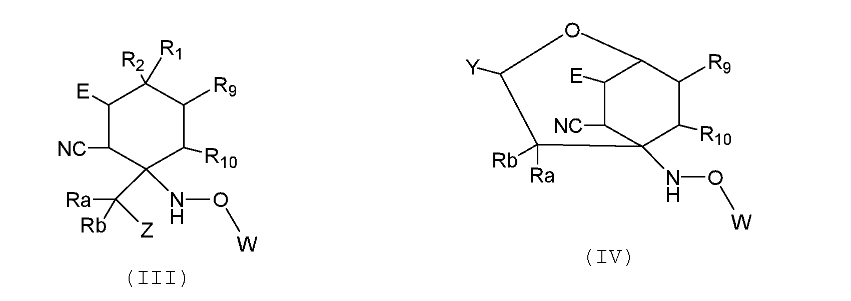 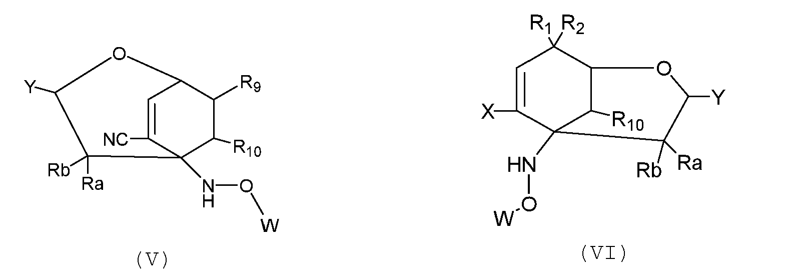 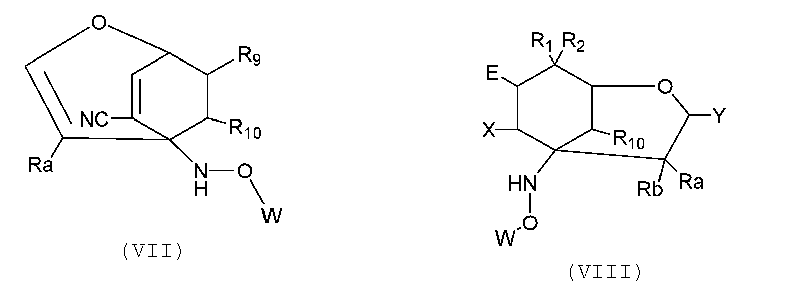 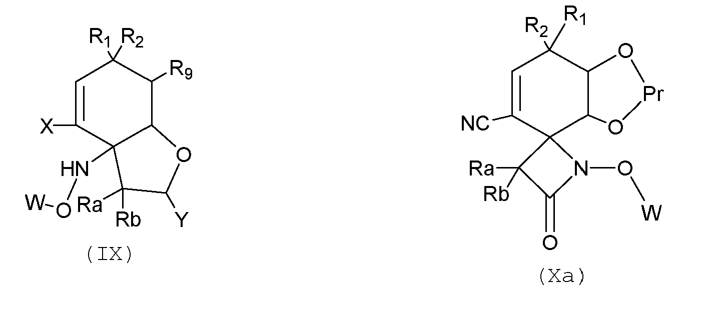 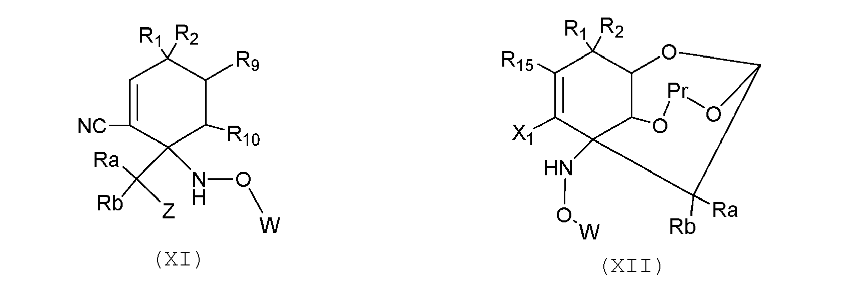 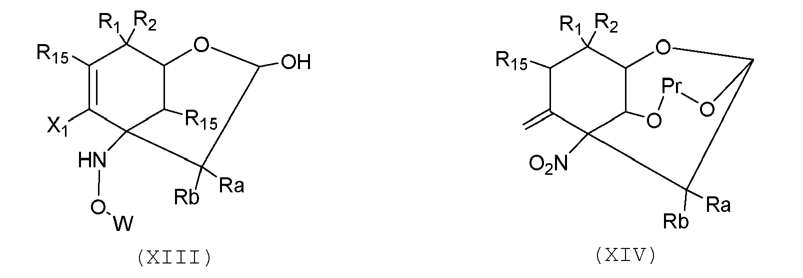 wherein
R₁ is selected from hydrogen, cyano, substituted or unsubstituted alkyl, substituted or unsubstituted alkenyl, substituted or unsubstituted alkynyl and NReRf, wherein Re and Rf are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxyl, substituted or unsubstituted aryloxyl, substituted or unsubstituted amino and halogen;
R₂ is selected from hydrogen, OH, or OPr; or
R₁ and R₂ together form a group selected from =O, alkylidene and -CH₂-O-Pr-O-;
E is selected from the group consisting of fluorine, chlorine, bromine, iodine, -SeR₁₃, -O-NR₁₃, -SR₁₄, PO(O-alkyl)₂ and PO(O-aryl)₂, wherein
R₁₃ is selected from the group consisting of aryl and alkyl;
R₁₄ is selected from the group consisting of aryl, alkyl, - PO(O-alkyl)₂, PO(O-aryl)₂, -C(=O)O-alkyl and -C(=O)O-aryl;
R₉ and R₁₀ are each independently selected from hydrogen, OH, OPr and =O; or together form a -O-Pr-O- group;
W is selected from the group consisting of -H, substituted or unsubstituted alkyl, substituted or unsubstituted aryl, substituted or unsubstituted arylalkyl, substituted or unsubstituted heterocyclyl and substituted or unsubstituted alkenyl;
Ra and Rb are each independently selected from H, OH, OPr, Se-aryl, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxyl, substituted or unsubstituted aryloxyl, substituted or unsubstituted amino and halogen;
Pr is a hydroxyl protecting group;
Z is -COOH or -CHR₄R₅, wherein R₄ is hydrogen, and R₅ is OH or OPr; or R₄ and R₅ together form =O;
X is -CH(=O) or -CN;
Y is selected from the group consisting of -OR₆, -SR₆, Se-aryl, -N(R₆)₂, -+N(R₆)₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl;
R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, - O-arylalkyl, O-(C=O)-alkyl, O-(C=O)-aryl and O-(C=O)- arylalkyl;
X₁ is selected from the group consisting of -CN, -COOR₁₆, - CON(R₁₆)₂, CHO, -CH₂OH, CH₂N(R₁₆)₂-Se-Aryl, -Se (=O) -aryl, said aryl being able to be optionally substituted, -S-R₁₆, -S(=O)R₁₆, - CH(OR₁₆)₂, -CH(NHR₁₆)CN and -CH(OR₁₆)CN
wherein R₁₆ is in each case independently selected from the group consisting of hydrogen, alkyl and aryl; two R16 groups being able to form a linear or branched alkylidene group, optionally substituted with one or more carbonyl groups; and
R₁₅ is independently in each case -OH or -OPr.

The compounds of the invention are intermediate products especially interesting for synthesizing TTX, providing a flexible pathway to its different analogs.

The invention also provides salts of the compounds of the invention with biologically and pharmacologically acceptable inorganic and organic acids, non-limiting examples of which are sulfates; halohydrate salts; phosphates; lower alkane sulfonates; arylsulfonates; salts of C₁-C₂₀ aliphatic mono-, di- or tribasic acids which can contain one or more double bonds, an aryl nucleus or other functional groups such as hydroxyl, amino, or keto; salts of aromatic acids in which the aromatic nuclei may or may not be substituted with groups such as hydroxyl, lower alkoxyl, amino, mono- or di-(lower alkyl)-amino-sulfonamido. There are also included within the scope of the invention quaternary salts of the tertiary nitrogen atom with lower alkyl halides or sulfates, and oxygenated derivatives of the tertiary nitrogen atom, such as N-oxides. The persons skilled in the art will select the pharmaceutically acceptable salts when preparing dosage formulations.

The term "solvate" according to this invention must be understood as meaning any form of active compound according to the invention which has another molecule (most likely a polar solvent) bound to it through a non-covalent bond. Examples of solvates include hydrates and alcoholates, e.g. methanolate.

The present invention also comprises the different stereoisomers of the compounds of the invention. A "stereoisomer" in the present patent application refers to compounds formed by the same atoms bound by the same bonding sequence but which have different three-dimensional structures that are not exchangeable.

Particular embodiments of the compounds of the invention are made up of the compounds of formula (IIa), (IIIb), (IIIc), stereoisomers, salts or solvates thereof 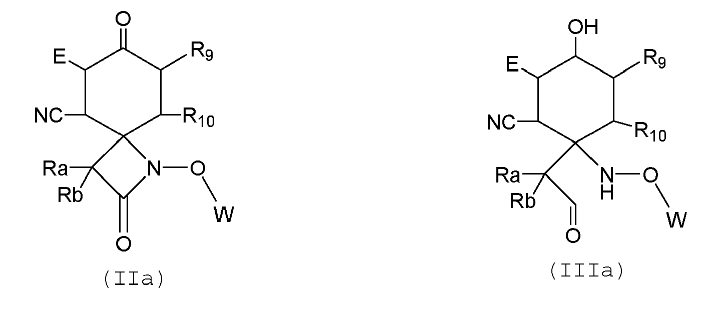 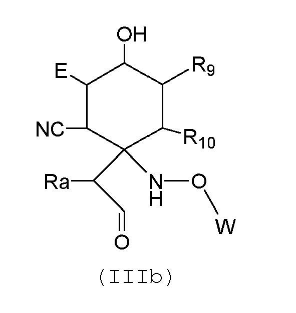 wherein E, R₉, R₁₀, Ra, Rb and W are as have been defined above.

According to a particular embodiment W is arylalkyl, preferably W is CRcRd-aryl, wherein Rc and Rd are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxyl, substituted or unsubstituted aryloxyl, substituted or unsubstituted amino and halogen. In a particular embodiment Rc is other than hydrogen and Rd is hydrogen. According to an additional embodiment Rc and Rd are both hydrogen.

According to a particular embodiment Ra is hydrogen and Rb is selected from the group consisting of halogen, preferably bromine, OH, OPr and Se-aryl. In another embodiment Ra and Rb are both hydrogen.

According to a particular embodiment, Y is selected from OH, alkoxyl, ammonium and -NH(O-(C=O)-aryl).

According to a particular embodiment, E is bromine or iodine, preferably bromine.

According to a particular embodiment, R₉ and R₁₀ together form a group of formula -O-Si(R₁₁R₁₂)-O-Si(R₁₁R₁₂)-O-, wherein R₁₁ and R₁₂ are independently selected from the group consisting of hydrogen, substituted or unsubstituted alkyl, substituted or unsubstituted cycloalkyl, substituted or unsubstituted alkenyl, substituted or unsubstituted alkynyl , substituted or unsubstituted aryl and substituted or unsubstituted heterocyclyl. According to a particular embodiment, R₁₁ and R₁₂ are isopropyl, for example in the compounds of formula (II), (III), (IV), (V), (VI), (VII), (VIII), (IX), (X), (Xa), (XI), (XII), (XIII) or (XIV).

According to a particular embodiment, R₁ is alkyl-OPr. According to another particular embodiment, R₁ and R₂ together form =O, alkylidene or -CH₂-O-Pr-O-. According to a particular embodiment, R₁ and R₂, in the compounds of formula (X), (Xa), (XI), (XII), (XIII) or (XIV), for example, together form -CH₂-O-Pr-O-, wherein Pr is -[(R₁₁)C(R₁₂)]-, wherein R₁₁ and R₁₂ are as have been defined above, preferably methyl. In another particular embodiment R₁ is hydrogen and R₂ is OH or OPr.

In a particular embodiment, X is CN.

### Synthesis of the compounds of the invention

An aspect of the present invention is a method for synthesizing a compound of formula (II), stereoisomers, salts or solvates thereof, comprising a sequence A which comprises (ai) reacting a compound of formula (I) in the presence of a cyanide ion source, and (aii) reacting the intermediate obtained in the presence of an electrophile, or a sequence B which comprises (bi) reacting a compound of formula (I) in the presence of electrophile, and (bii) reacting the intermediate obtained in the presence of a cyanide ion source 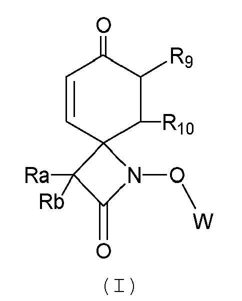 wherein R₉, R₁₀, Ra, Rb and W are as have been defined above.

The methods for obtaining the compounds of formula (I), starting materials of the present invention, are described in patent applications PCT/EP2005/005149 and PCT/EP2005/005146, of the same inventors.

In Sequence A, step (ai) provides a Michael addition on position 4a of the cyclohexenone ring of the compound of formula (I). The intermediate enolate of formula (Ia) is produced as a result of said attack.

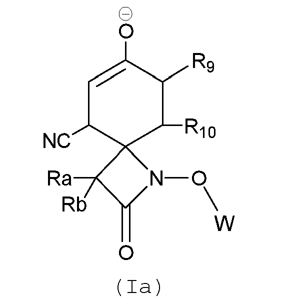

The electrophile can be added directly in the reaction medium after the reaction with cyanide ion source, without the need of isolating said intermediate enolate of formula (Ia). Alternatively, said enolate can be trapped and isolated before its reaction with the electrophile.

In sequence B, the electrophile is first added to provide the corresponding derivative of position 5 (Ib)

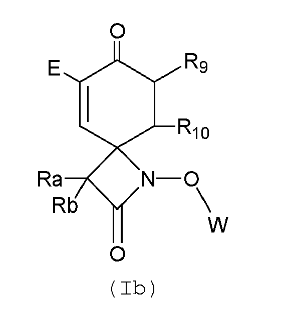

Michael addition on said derivative is then performed with the cyanide ion. According to a particular embodiment, the derivative of formula (Ib) is isolated and then subjected to reaction (bii) in the presence of a cyanide ion source. According to a particular embodiment, the reaction (bi) is also performed in the presence of a base, for example, an organic base such as a trialkylamine (e.g. triethylamine).

Alternatively, the compounds of formula (I) can be reacted in the presence of a cyanide ion source and, instead of reacting the intermediate obtained in the presence of an electrophile, the intermediate enolate can be reacted with a proton source. Compounds of formula (X), stereoisomers, salts or solvates thereof are obtained as a result. 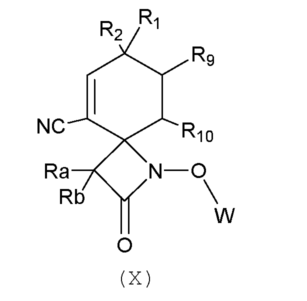 wherein R₁, R₂, R₉, R₁₀, Ra, Rb and W are as defined above.

The different definitions for R₁ and R₂ radicals in the compounds of formula (X) can be obtained by following the methods described in the section "Other Reactions" below.

Of these compounds, the compounds of formula (Xa) described above are a novel subgroup especially useful for synthesizing the TTX. As can be seen, these compounds of formula (Xa) allow arriving, in a few steps, to the compounds of formula (XI), (XII), (XIII) or (XIV), all of them are also novel advanced intermediates for synthesizing TTX compounds.

In another embodiment, the compounds of formula (X) can be obtained by means of sequence A giving rise to the compounds of formula (II), followed by treatment with base (for example, a trialkylamine such as triethylamine) giving rise to the removal of group E. For example, it is possible to treat a compound of formula (Ia) with NBS to produce the resulting compound of formula (II) wherein E is bromine, which compound can be converted into a compound of formula (X) by means of treatment with triethylamine.

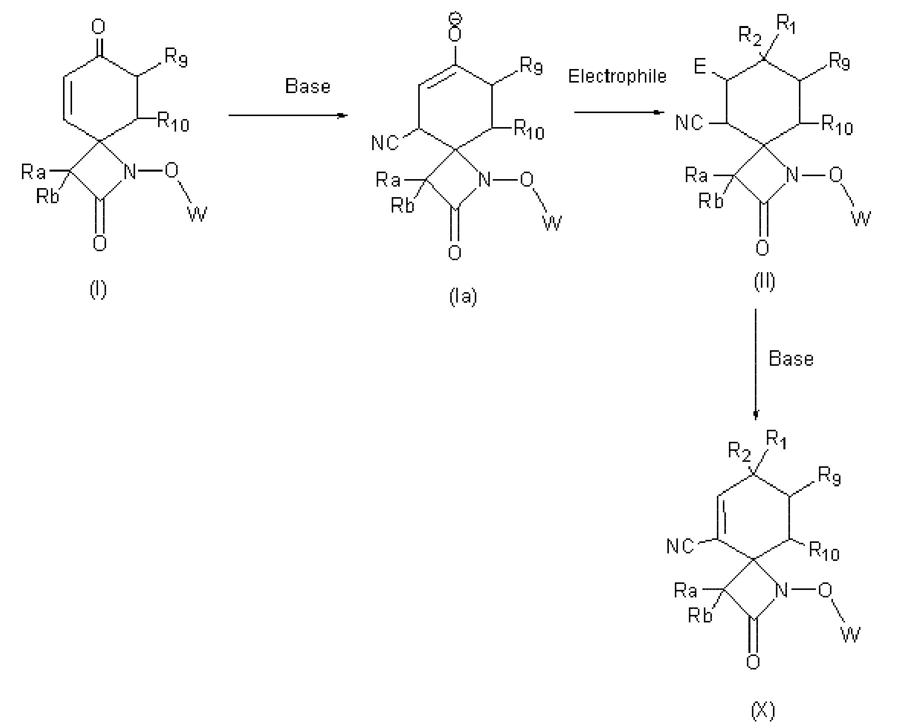

According to a particular embodiment, Pr in a compound of formula (Xa) is -O-Si(R₁₁R₁₂)-O-Si(R₁₁R₁₂)-O-, wherein R₁₁ and R₁₂ are as have been defined above, wherein R₁₁ and R₁₂ are preferably isopropyl. In a compound of formula (Xa) at least one of Ra and Rb is preferably hydrogen.

The cyanide ion source necessary can be obtained from different sources known by the person skilled in the art. For example, said sources can be selected from the following reactive systems: Ph₃P/diethyl azodicarboxylate (DEAD)/alcohol/acetone cyanohydrin (Synth. Com. 1995, 25, 1545-1550; Synth. Com. 1993, 23, 2481-2484).

Other cyanide anion sources are based on reactive systems comprising NaCN. The conditions already described in the art comprise a mixture of NaCN with one of the following reaction systems: DMSO or nBu₄NCN/DMF/heat (J. Med. Chem. 1991, 56, 3009-3016; J. Med. Chem. 1966, 31, 2933-2941), DMSO/heat (J. Med. Chem. 2001, 44, 94-104; Tetrahedron Asymmetry, 1996, 7, 1967-1972), Bu₄NCN/DMSO/heat (Bioorg. Med. Chem. Lett. 1999, 9, 841-846), DMF/heat (Tetrahedron Letters, 1998, 39, 3357-3358; J. Med. Chem. 1994, 37, 4195-4210), [(CH₃)₂N]₃PO (J. Chem. Soc. Chem. Com. 1982, 7, 404-406) or conditions in which sodium bicarbonate is present, such as NaCN/NaHCO₃/H₂O/Et₂O/r.t., NaCN/NaHCO₃/Et₂O/r.t. or NaCN/NaHCO₃/THF/-H₂O (J. Med. Chem. 1992, 35, 2721-2727; Carbohydrate Research 1991, 216, 399-411; J. Med. Chem. 1998, 41, 4636-4647; Carbohydrate Research 1991, 216, 399-411). Also, NaCN in the presence of 1-methyl-1H-pyrrole provides the desired cyanide anion.

In another embodiment, the reaction can be performed in the presence of KCN (J. Am. Chem. Soc. 1958, 80, 4677-4680). The conditions already described in the art comprise a mixture of KCN with one of the following reaction systems: Et₂O/H₂O (Tetrahedron Letters, 2001, 42, 6259-6262), THF/r.t. (Carbohydrate Research 1994, 254, 133-140) or CH₂Cl₂/H₂O/r.t., with or without NaHCO₃ (J. Chem. Soc. Perkin Trans 1. 1985, 1067-1072).

In another embodiment, the reaction can be performed in the presence of LiCN. The conditions already described in the art comprise a mixture of LiCN with one of the following reaction systems: Me₃SiCl/THF/r.t. (Synthesis, 1986, 12, 1054-1055), DMF/[(CH₃)₂N]₃PO (Bioorg. Med. Chem. Lett. 1996, 6, 1897-1900), diethyl cyanophosphonate/THF/r.t. (Chem. Pharm. Bull. 1986, 34, 4620-4628; Tetrahedron Letters, 1989, 30, 3681-3684) or DMF (Bioorg. Med. Chem. Lett. 2000, 10, 2417-2419).

Copper cyanide is also a useful cyanide anion source (CuCN/HNaCO₃/H₂O/EtO₂; Tetrahedron. 1988, 44, 4895-4904).

In another embodiment, the reaction can be performed in the presence of HCN. The conditions already described in the art comprise a mixture of HCN with one of the following reaction systems: Pyridine/r.t. (J. Chem. Soc. Perkin Trans 1. 1994, 1067-1072), Et₃N/CH₂Cl₂/0°C (Carbohydrate Research 1986, 155, 236-246) or the in situ generation of HCN mixing Zn(CN)₂ and AlCl₃ (Tetrahedron Asymmetry, 1990, 1, 187-198).

Therefore, according to an embodiment, the reagent providing a cyanide anion has a formula MCN, wherein M is lithium, potassium, sodium or copper.

Trialkylsilyl cyanides are also very widespread cyanide anion sources (J. Org. Chem. 2003, 3094-3103; J. Am. Chem. Soc. 1973, 5822-5823; J. Chem. Soc. Perkin Trans 1. 1988, 2305-2307). The conditions already described in the art comprise a mixture of trialkylsilyl cyanide with one of the following reaction systems: TMSCN/DABCO or TMSCN/(DHQ)₂AQN (J. Am. Chem. Soc. 2003, 125, 9900-9901), TMSCN/BF₃/CH₂Cl₂/r.t. or TMSCN/BF₃·Et₂O/CH₂Cl₂/r.t. (Tetrahedron Letters, 1990, 31, 71-74; Tetrahedron Letters, 1990, 31, 71-74; Tetrahedron Letters, 1990, 31, 71-74; Carbohydrate Research 1994, 258, 77-86), TMSCN/crown ether/KCN(cat)/toluene/-30°C (J. Am. Chem. Soc. 1985, 107, 4577-4579; Tetrahedron Letters, 1994, 35, 7901-7904), TMSCN/amine/CH₂Cl₂/0°C (Chemistry Letters, 1991, 125, 537-540), TMSCN/I₂/20-30°C (Tetrahedron 2000, 35, 6533-6540), TMSCN/ZnI₂/heat (Tetrahedron Asimmetry. 1994, 9, 805-816), TMSCN/CaO/Toluene/-40°C (J. Chem. Soc. Chem. Com. 1991, 15, 1035-1036), TMSCN/Ph₃P(cat)/CH₂Cl₂ (Tetrahedron Letters, 1986, 27, 2757-2760), TMSCN/Lewis acid (Tetrahedron Letters, 1983, 39, 967-974), TMSCN/catalyst (Tetrahedron 2001, 57, 771-779); TMSCN/ZnI₂(cat) (Tetrahedron 1994, 50, 2821-2830), TBDMSCN/ZnI₂/CH₂Cl₂ (J. Org. Chem. 1993, 58, 159-164; Bull. Chem. Soc. Jpn. 2001, 74, 997-1008), (alkyl)₃SiCN/LiCl(cat)/without solvent (J. Org. Chem. 2005, 70, 6530-6532).

According to an additional embodiment, the reagents providing a cyanide anion are a dialkylaluminium cyanide of formula (alkyl)₂-Al-CN or a trialkylsilyl cyanide of formula (alkyl)₃Si-CN, which are available on the market.

According to a preferred embodiment, the reaction proceeds in the presence of TMSCN or Et₂AlCN.

According to a particular embodiment, electrophiles useful for the preparation of the compounds of formula (II) are selected of the group consisting of R₁₃S-SR₁₃, for example, Me-S-S-Me (Yadav, V. K.; Babu, K. G.; Parwez, M. J. Org. Chem. 2004, 69, 3866-3874), or Ph-S-S-Ph (Morel, G.; Marchand, E.; Foucaud, A. Synthesis 1980, 11, 918-921); R₁₃-S-SO₂-R₁₃, for example, Ph-S-SO₂-Ph (Lythgoe, B.; Waterhouse, I.; J. Chem. Soc. P. T. 1 1979, 2429-2436); Cl-S-PO(O-alkyl)₂, for example, Cl-S-PO(OEt)₂ (Dybowski, P.; Skowronska, A. Tetrahedron Lett. 1991, 32, 4385-4388); Cl-S-C(=O)O-alkyl, for example, MeO-CO-S-Cl (Sanemitsu, Y.; Kawamura, S.; Tanade, Y. J. Org. Chem. 1992, 57, 1053-1056); Cl-PO(O-alkyl)₂, for example, Cl-PO(OEt)₂ (Kandil, A. A.; Porter, T. M.; Slessor, K. N. Synthesis 1987, 4, 411-413); ONR₁₃, for example, Ph-NO (Momiyama, N.; Yamamoto, H.; Org. Lett. 2002, 21, 3579-3582); BrSeR₁₃, for example, BrSePh (Rollinson, S. W.; Amos, R. A.; Katzenellenbogen, J. A. J. Am. Chem. Soc. 1981, 103, 4114-4125), or BrSeMe (Aoki, I.; Nishibayashi, U.; Sakae, U. Bull. Chem. Soc. Japan 1995, 68, 337-340); R₁₃Se-SeR₁₃, for example, Ph-Se-Se-Ph (Wovkulich, P. M.; Baggiolim, E. G.; Hennessy, B. M.; Uskokovic, M. R. Heterocycles 1993, 35, 791-806). Those mentioned between parenthesis indicate a reference in which possible reaction conditions for using the reagents are described. Other conditions can be found in reference books such as *"*Advanced Organic Chemistry: reactions, mechanisms and structures", March, J., fourth edition, Wiley-interscience.

Thus, the different intermediate compounds of the invention and the starting materials can be prepared using a basic set of simple reactions which allow specifically protecting and functionalizing the different positions. These methods will be explained below. Throughout the description and claims the configurations given are solely relative configurations.

The enantiomerically pure derivatives of the compounds of the invention can be prepared using starting materials or reagents (for example, Sharpless dihydroxylation or epoxidation of a double bond) having the opposite optical rotation. Furthermore, the conventional techniques for separating diastereoisomers and enantiomers are generally known by the persons skilled in the art.

As has been mentioned above, the starting materials of the present invention can be obtained by methods described in documents PCT/EP2005/005149 and/or PCT/EP2005/005146. The key intermediate products described in document PCT/EP2005/005146 are benzodienones with the following formula 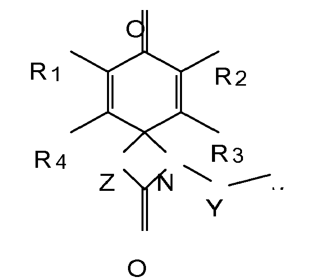 wherein the substitution in position Z can create a stereogenic centre. In document PCT/EP2005/005146 a preferred embodiment is that wherein Z is -CRaRb-, wherein Ra and Rb are different and thus create a chiral centre. Thus, according to a preferred embodiment Ra and Rb in the compounds of the invention are different, and a chiral centre is thus created.

Furthermore, document PCT/EP2005/005146 describes as a preferred embodiment compounds wherein the substituent W of the benzodienones above is an arylalkyl group, more preferably wherein W is -CRaRb-Q, wherein Ra and Rb in the connector essentially have the same meaning as in the present application, and the substituent Q has π (pi) interactions with those moieties of benzodienone. Thus, another chiral source arises when Ra and Rb in said connector are different, preferably wherein W is -CHRa-Q (e.g., (-)-(S)-1-(1-phenylethoxy)-1-azaspiro[3.5]nona-5,8-diene-2,7-dione, which is described as compound 5d in document PCT/EP2005/005146). As described in document PCT/EP2005/005146 said configurations provide a stereogenic centre allowing the selectivity or specificity of any other reaction, distinguishing the two double bonds of the benzodienones mentioned, which are intermediate products in the synthesis of the compounds of the invention. This will open up a pathway to diastereoselective and enantioselective synthesis.

Therefore, by using the chiral materials described in documents PCT/EP2005/005149 and/or PCT/EP2005/005146, the specific stereoisomers and enantiomers of the compounds described in the present invention can be obtained.

To obtain the different compounds of formula (II) or of formula (X) or of formula (XI), the latter or the precursors thereof can be subjected to one or more steps which are selected from the group consisting of:
a) reducing the ketone to hydroxyl group, optionally followed by protecting and/or inverting the configuration of said hydroxyl group;
b) olefinating the ketone to obtain an alkylidene group, optionally followed by dihydroxylation;
c) olefinating the ketone to obtain an alkylidene group, optionally followed by dihydroxylation and subsequent protection of at least one of the hydroxyl groups formed;
d) protecting the ketone;
e) alkylating the ketone; and
f) aminating the ketone.

The ketone of a compound of formula (IIa) for example can be reduced to hydroxyl and to subsequently protect it, or it can be subjected to a Wittig reaction to obtain methylene derivative. The ketone can also be protected (see scheme 2)

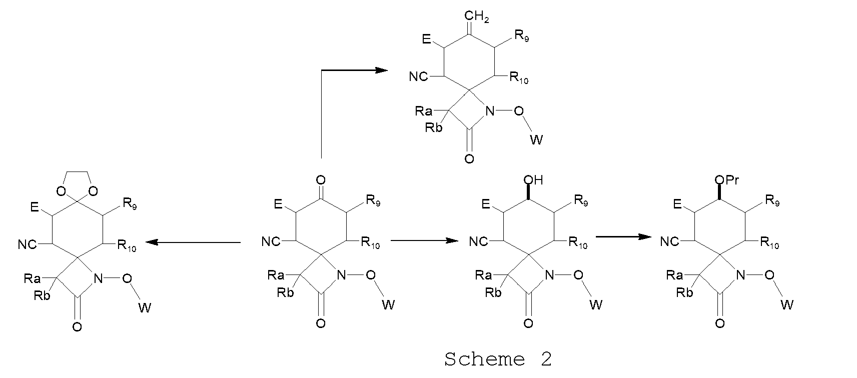

Another aspect of the present invention is a method for synthesizing a compound of formula (III) or (XI), stereoisomers, salts or solvates thereof, which comprises reacting a compound of formula (II) or (X), respectively, in the presence of a hydride. The spirolactam ring is opened by means of this reaction, carbon 10 being able to obtain (according to the TTX numbering) either aldehyde or alcohol depending on the nature of the hydride and of the reaction conditions (for example, by means of choosing the solvent or the number of hydride equivalents). Optionally, the resulting hydroxyl can be protected. Another alternative is oxidizing the aldehyde or alcohol obtained to obtain the corresponding carboxylic acid.

The spirolactam ring is opened in the presence of a hydride, preferably in the presence of an aluminium or boron hydride derivative, for example, 9-BBN, NaBH₄, DIBALH or LiAlH₄ (see for example chapter 19 of "Advanced Organic Chemistry: reactions, mechanisms and structures", March, J., 5th edition, Wiley-interscience). Other non-limiting hydrides and methods suitable for the present invention are described in Handbook of Reagents for Organic Synthesis. Oxidizing and Reducing Agents. Eds; John Wiley & Sons: Baffins Lane, Chichester, 1999: diborane (pages 126-132), BH3·SMe2 (pages 48-51), BH3·THF (pages 52-57), ((CH3)2-CH(CH3))2BH (pages 160-163) or diisopinocamphenylborane (pages 146-149).

An additional aspect of the invention is a method for synthesizing a compound of formula (IV), stereoisomers, salts or solvates thereof, which comprises
(a) reacting a compound of formula (IIIa) in the presence of a base.
   or,
(b) when the compound of formula (IV) wherein Ra is -Se-aryl is to be obtained, reacting a compound of formula (IIIb) in the presence of halogen-Se-aryl or aryl-Se-Se-aryl, and a base, preferably a cyclic amine, preferably morpholine.

Methods (a) and (b) for obtaining compounds of formula (IV) can also be used to obtain the compounds of formula (V), depending on the base used. This also forms additional aspects of present invention. In some cases the compounds of formula (V) or the compounds of formula (IV) are exclusively obtained, and in other occasions mixtures of both (see examples 7 and 8 below).

The compounds of formula (V), for example, formally result from the removal of HBr from a compound of formula (IV). Therefore, harsher conditions (higher temperature, stronger bases, excess of bases, greater reaction times, etc.) would favor the formation of compounds of formula (V). As seen by comparing Examples 7 and 8, a more prolonged exposure to the base (example 8) and/or the excess thereof favors the removal and therefore the formation of the compounds of formula (V). In addition, weaker bases cannot perform the removal and they favor the formation of compounds of formula (IV). Other conditions which can favor the formation of compounds of formula (IV) are the use of less base and shorter reaction times.

According to a particular embodiment, the base selected from the group consisting of phosphazenes (P1, P2, P3 and P4) and related bases such as Verkade's base; DBU; DABCO; cyclic aliphatic amines such as pyridine, 4-dimethylaminopyridinpiperidin or morpholine; trialkylamines such as triethylamine or the diisopropylamine. According to a particular embodiment, said amines are supported on polymer supports. According to a particular embodiment the base is DBU.

In a particular embodiment, the compound of formula (V) or of formula (IV) obtained is reacted in an acid medium in the presence of a reagent which is selected from the group consisting of HOR₆, HSR₆, Se-aryl, aryl-Se-Se-aryl, HN(R₆)₂, N(R₆)₃ and -NHR₇,
wherein
R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and
R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, - O-arylalkyl, O-(C=O)-alkyl, O-(C=O)-aryl and O-(C=O)- arylalkyl.

This embodiment allows exchanging group Y to then obtain different derivatives. The nature of said acid medium is not of special importance and any acid system is suitable. Non-limiting examples of substances which can give rise to an acid medium are p-toluenesulfonic acid (p-TsOH) or the like (for example, camphorsulfonic acid), inorganic acids such as hydrochloric acid or sulfuric acid, or acid materials such as some zeolites. Said acids can be supported on polymer supports.

An additional aspect of the present invention is a method for obtaining a compound of formula (VI), stereoisomers, salts or solvates thereof, which comprises reacting a compound of formula (V) in an acid medium in the presence of a reagent which is selected from the group consisting of HOR₆, HSR₆, Se-aryl, aryl-Se-Se-aryl, HN(R₆)₂, N(R₆)₃ and -NHR₇, wherein R₆ and R₇ are as have been defined above.

This reaction can also produce compounds of formula (IX), stereoisomers, salts or solvates thereof, a method which also forms an aspect of the invention. See Example 11 below.

Another aspect of the present invention is a method for obtaining a compound of formula (VII), stereoisomers, salts or solvates thereof, which comprises reacting a compound of formula (V) with a base. Said reaction involves the removal of group Y, and it will therefore be more effective the better the leaving group Y is. In a particular embodiment, the reaction is performed on a compound of formula (V) wherein Y is an ammonium group, i.e., a group of formula -+N(R₆)₃.

An additional aspect of the present invention is a method for obtaining a compound of formula (VIII), stereoisomers, salts or solvates thereof, which comprises reacting a compound of formula (IV) in an acid medium in the presence of a reagent which is selected from the group consisting of HOR₆, HSR₆, Se-aryl, aryl-Se-Se-aryl, HN(R₆)₂, N(R₆)₃ and -NHR₇, wherein R₆ and R₇ are as have been defined above.

In a particular embodiment, the methods described also comprise the simultaneous deprotection of R₉ and R₁₀.

In a particular embodiment, the methods described comprise introducing a -Se-aryl group, preferably by means of using phenylselenium bromide in position 9, and optionally its subsequent reaction with a group of formula OR₈, wherein R₈ is selected from the group consisting of -(C=O)R₆ and -NHR₆, wherein R₆ is as has been defined above.

An additional aspect of the invention is made up of a method for synthesizing a compound of formula (XII) which comprises reacting a compound of formula (XI) in the presence of a base. According to a particular embodiment and similarly with the synthesis of the compounds of formula (IV), the base can be selected from the group consisting of phosphazenes, DBU, DABCO, cyclic aliphatic amines and trialkylamines, preferably DBU.

An additional aspect of the invention is made up of a method for synthesizing a compound of formula (XIII), which comprises deprotecting the hydroxyls of positions 8 and 10, according to the TTX numbering, removing the Pr group of a compound of formula (XII). For the cases wherein Pr is a silicon-based protective group, for example -Si(R₁₁R₁₂)-O-Si(R₁₁R₁₂)-, the deprotection can be carried out with a fluoride source or another reagent as described in textbooks (e.g. Greene and Wuts "Protective Groups in Organic Synthesis", John Wiley & Sons, Inc., New Jersey, 2007). An example of this reaction comprises Example 27 below.

An additional aspect of the invention comprises a method for synthesizing a compound of formula (XIV), which comprises reacting a compound of formula (XII), wherein X₁ is -Se-aryl or -SR₁₆, in the presence of a peroxide, preferably in the presence of peroxide hydroxide. Corresponding oxidized selenium (-Se(=O)-aryl) or sulfur (-S(=O)-R₁₆) derivative is formed under these conditions giving rise to transposition in which the double bond between positions 4a and 5 (according to the TTX numbering) takes part and which after hydrolysis provides the compound of formula (XIV). The conditions for performing this type of transformations are known in the state of the art (see for example Scianowski, J. Tetrahedron 2009, 65, 10162-10174; Toyofuku, M. Org. Let. 2008, 10, 3957-3960; Oshida, M. Chem. Pharm. Bull. 2008, 56, 404-406, for the case of selenium, or Braveman, S. Rearragements Involving Sulfoxides The Chemistry of Sulphones and Sulphoxides, Chapter 14; Ed. John Wiley&Sons, 1988; p. 717-757, for the case of sulfur.

Therefore, another aspect of the invention is a method for synthesizing TTX which comprises transforming a compound of formula (XII) in a compound of formula (XIII) or of formula (XIV), according to the conditions described above. In a preferred embodiment, said compound of formula (XII) is obtained from a compound of formula (XI) according to the conditions described above. In another particular embodiment, said compound of formula (XI) is obtained from the compound of formula (X) according to the conditions described above.

### Other Reactions

The compounds of the invention can comprise different hydroxyl groups, the configuration of which can be inverted by means of a Mitsonobu reaction. For example see (Mitsunobu, 0.; Synthesis, 1, 1981)

The compounds of formula (II), (III), (VI), (VIII), (IX), (Xa), (XI), (XII), (XIII) or (XIV) can give different combinations for the groups R₁ and R₂, all of them can be obtained by means of the reaction sequence described in PCT/EP2005/005146 and PCT/EP2005/005149, the content of which is incorporated by reference. For example, see scheme 3.

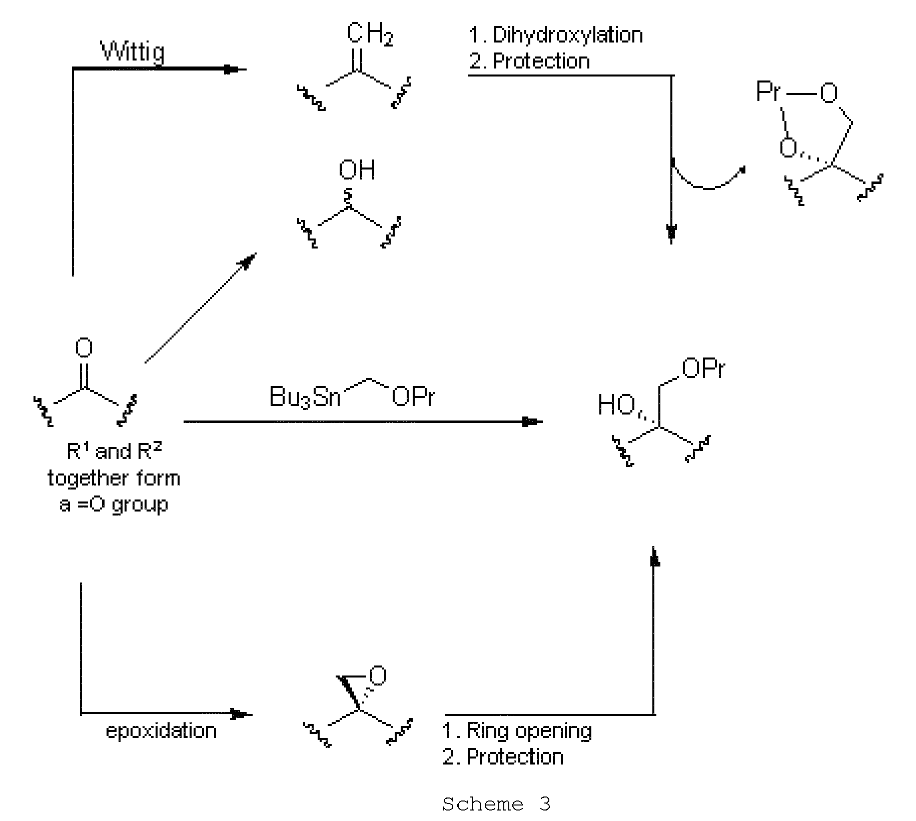

The presence of vicinal hydroxyl groups allows the simultaneous protection of two or more hydroxyls. This can be achieved by means of using acetals, such as acetals of isopropylidene, cyclohexylene, cyclopentenyl, arylmethylene, diphenylmethylene, 1,2-diacetals such as dispiroketals, cyclohexane-1,2-diacetals, butane-2,3-diacetals, silylenes, 1,1,3,3-tetraisopropyldisiloxanylidenes or N,O-acetals. Additional examples of diol protecting groups can be found in reference books such as Greene and Wuts' "Protective Groups in Organic Synthesis", John Wiley & Sons, Inc., New York, 1999. Borolanes can also be formed on two vicinal hydroxyl groups, for example by using phenyl boronic acid.

Once the compounds of formula (VI), (VIII) or (IX) wherein X is -CN are obtained, it is easy to obtain the corresponding aldehyde by reduction with DIBAL, for example (see example 13).

Similarly, -CN (position 4 according to the TTX numbering) in the compounds of formula (XII) or (XIII) can be converted into different functional groups such as -COOR₁₆, -CON(R₁₆)₂, CHO, -CH₂OH, CH₂N(R₁₆)₂, -Se-Aryl, -Se(=O)-aryl, said aryl being able to be optionally substituted, -S-R₁₆, -S(=O)R₁₆, -CH(OR₁₆)₂,-CH(NHR₁₆)CN and -CH(OR₁₆)CN. The conditions for said conversions are known by the person skilled in the art. For example, aldehyde (-CH(=O)) can be obtained by reducing the nitrile group with DIBAL, as mentioned in the paragraph above. Alternatively, the -CH₂OH group can be obtained by aldehyde reduction in the presence of a reducing agent, either from the aldehyde or directly from the nitrile. In addition, the (-COOH) acid can be obtained by oxidizing the nitrile, aldehyde or -CH₂OH group, which can in turn be converted into different acid derivatives such as esters (-COOR₁₆), amides (-CONR₁₆). The corresponding - CH₂OH groups can also be converted into amines or thiols, or the aldehydes to be protected. The conditions for carrying out all these reductions are known by the person skilled in the art and are described in reference books such as chapter 19 of "Advanced Organic Chemistry: reactions, mechanisms and structures", March, J., 5th edition, Wiley-interscience, for example.

A -Se-aryl or -SR₁₆ group from the corresponding aryl can also be obtained by following the known methods. For example, reacting the corresponding hydroxyl with a compound of formula L-Se-aryl in the presence of a base. The group L is a leaving group, for example, nitrile or isoindole-1,3-dione. The bases can be amines or trialkylphosphorus, for example, n-Bu₃P. More conditions which can give rise to this conversion can be found for example in Swamy, K.C.K.; Kumar, N.N.B.; Balaraman, E.; Kumar, K.V.P.P. Chem. Rev. 2009, 109, 2551-2651; Brian, A.; Sivaramakrishhnan, A.; Naka, T.; Koide, K. J. Am. Chem. Soc. 2006, 128, 2792-2793; Brian, A.; Sivaramakrishhnan, A.; Naka, T.; Koide, K. J. Am. Chem. Soc. 2007, 129, 2648-2659; Bourland, T.C.; Carter, R.G.; Yokochi, A.F.T. Org. Biomol. Chem. 2004, 2, 1315-1329. The -SR16 group can also be introduced according to the conditions which are described in Umezawa, T.; Hayashi, T.; Sakai, H.; Teramoto, H.; Yoshikawa, T.; Izumida, M.; Tamatani, Y.; Hirose, T.; Ohfune, Y.; Shinada, T. Org. Lett. 2006, 8, 4971-4974; or in Tsay, S.-C.; Lin, L.C.; Furth, P.A.; Shum, C.C.; King, D.B. Synthesis 1993, 329-334; or in Karikubo, T.; Ogasawara, K. J. Chem. Soc. Chem. Commun. 1995, 1951-1952.

The invention will be illustrated in more detail by means of the examples which must not be interpreted as limiting to the scope of the claims.

### Examples

### Abbreviations and acronyms (obtained from Guide for Authors; Abbreviations and Acronyms J. Org. Chem. 2008, 78, 10A)

| | | | |
|---|---|---|---|
| Ac: | acetyl | M⁺: | molecular peak |
| Bn: | benzyl | Me: | methyl |
| Bu: | butyl | mg: | milligram |
| cat.: | catalytic | ml: | milliliter |
| ¹³C-NMR | carbon nuclear | mmol: | millimole |
| magnetic resonance | | m/z: | mass/charge ratio |
| d: | doublet | P.f.: | melting point |
| DBU: | 1,8-diazabicyclo[5.4.0]undec-7-ene | ppm: | parts per million |
| | | Ph: | phenyl |
| dd: | doublet of doublets | Pyr: | pyridine |
| ddd: | doublet of doublet | Yld.: | yield |
| of doublets | | s: | singlet |
| DMF: | N, N-dimethylformamide | sat.: | saturated aqueous solution |
| DMSO: | dimethylsulfoxide | t: | triplet |
| dt: | doublet of triplets | r.t.: | room |
| Et: | ethyl | temperature | |
| g: | gram | TBAF: | tetrabutylammonium |
| Hz: | hertz | fluoride | |
| IR: | infrared | TBDMS: | *tert*- |
| spectroscopy | | butyldimethylsilyl | |
| *J*: | coupling constant | TfO: | trifluoromethanesulfonate |
| LRMS: | low resolution mass | THF: | tetrahydrofuran |
| spectroscopy | | TMS: | trimethylsilyl |
| m: | multiplet | Ts: | *p*-toluenesulfonyl |
| M: | molarity | (tosyl) | |
| mM: | millimolar | *p*-TsOH: | *p*-toluenesulfonic |
| | | acid | |

### Naming and Numbering

The cyclohexane type structures will be named in the examples shown below as 2,3-dihydroxy-1-aminocyclohexane derivatives and they will be numbered according to the numbering indicated below, regardless of the substituent moiety which the molecule has in any position.

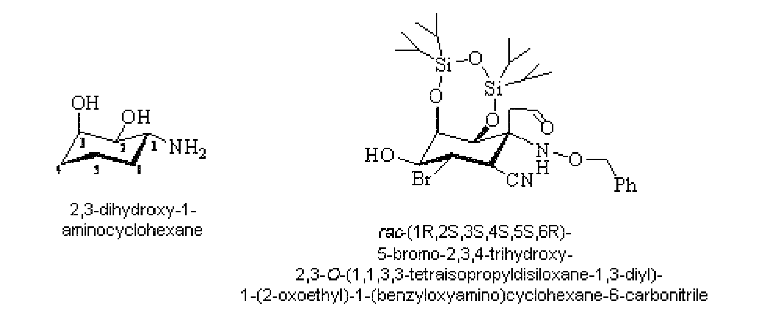

The non-spiranic bicyclic structures synthesized will be named and numbered as indicated below, which does not coincide with the rules established by the IUPAC

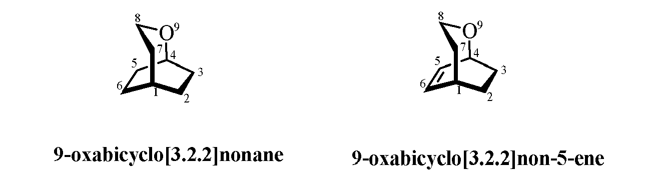

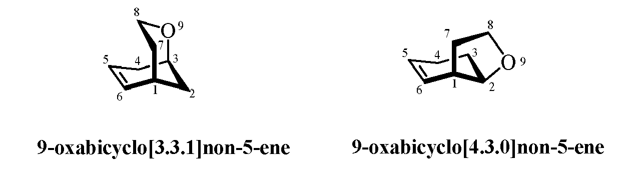

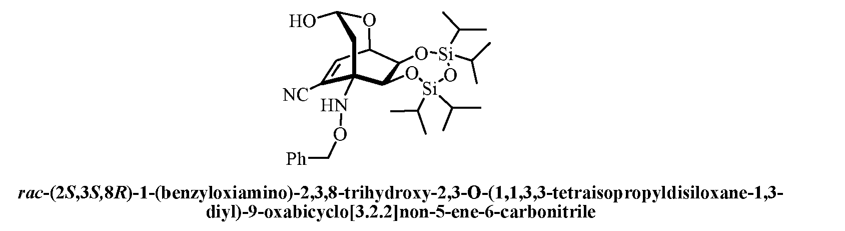

Unless otherwise indicated, the chiral products synthesized are racemic (rac) and will be graphically depicted by means of the figure of one of their enantiomers.

### Materials and Methods

All the reactions were performed under argon atmosphere, except those indicated in each case. The reagents and solvents used come from the commercial manufacturers Aldrich, Fluka, Merck, Sigma, Acros, Lancaster, SDS or Scharlau, and were purified by common methods (see Armarego, W. L.; Perrin, D. D. Purification of Laboratory Chemicals; Butterworth-Heinemann: Oxford, 1996.) when necessary.

The solvents used were distilled and dried under argon atmosphere as indicated below: CH₂Cl₂, toluene, benzene, DMSO and DMF on CaH₂ (subsequently dry benzene and DMF marketed by Aldrich and Fluka, respectively, were used); THF (pre-drying with KOH) and Et₂O on Na/benzophenone; CH₃CN, EtOH and MeOH on molecular sieve of 4Å in pore diameter (previously activated at 150°C). The CCl₄ marketed by Merck was used without distilling. The dioxane was degasified by passing an argon stream before being used.

The Et₃N, i-Pr₂EtN, i-Pr₂NH and pyridine were distilled under argon atmosphere on CaH₂. n-BuLi marketed by Aldrich was used as a 1.6 M solution in hexanes. 57-80% by weight m-CPBA marketed by Aldrich was used considering that its purity was 57%. NaH (60% in mineral oil) was washed twice with hexane under argon atmosphere immediately before being used. The Celite used was Celite-545 marketed by SDS.

DBU-polimer bound marketed by Aldrich with reference 595128 was used.

The reaction products were purified by low-pressure column chromatography (flash chromatography), using 60 Merck silica gel (with a 230-400 mesh particle size) as stationary phase and previously distilled solvents as mobile phase. The eluent used is indicated in each case and the ratios of the mixture of solvents used are always volume/volume.

The reactions were monitored by thin layer chromatography (TLC), using 60 F₂₅₄ silica gel chromatography plates marketed by Merck. The plates were developed using iodine vapors, 2% solution of 2,4-dinitrophenylhydrazine in EtOH (with 0.04% by volume of 97% H₂SO₄), 10% solution of phosphomolybdic acid in EtOH and UV light viewer (254 and 366 nm).

The (completely decoupled) ¹H and ¹³C nuclear magnetic resonance spectra were performed at room temperature in the solvent indicated in each case (CDCl₃, CD₃OD and DMSO-d₆) using the following apparatuses: Varian Gemini-200 (200 MHz), Varian INOVA-300 (300 MHz), Bruker Avance-300 (300 MHz) and Varian INOVA-400 (400 MHz). The chemical shift values are expressed in parts per million (δ, ppm), using as the internal reference the residue signal of the solvent: CDCl₃, 7.26 ppm (¹H-NMR) and 77.0 ppm (¹³C-NMR); CD₃OD, 3.31 ppm (¹H-NMR) and 49.0 ppm (¹³C-NMR).

The ¹H-NMR spectra are described indicating the number of protons and the apparent multiplicity of each signal. The coupling constants (J) are those apparent and are expressed in Hz. The following abbreviations have been used: s (singlet), d (doublet), t (triplet), c (quadruplet), q (quintuplet) and m (multiplet).

The assignment of NMR signals are provided in the double resonance and two-dimensional experiment techniques: DEPT (Distorsionless Enhancement by Polarization Transfer), HMBC (Heteronuclear Multiple-Bond Connectivities), HSQC (Heteronuclear Single Quantum Correlation) and nOesy (nuclear Overhauser effect spectroscopy).

The melting points (P.f.) were measured in a Reichert brand Kofler microscope.

The infrared spectra (IR) were recorded in the Perkin-Elmer spectrophotometer models 681 and FT-IR Spectrum One, and the frequencies (v) of the absorption maxima are expressed in cm⁻¹. The samples were analyzed as films between NaCl crystals or in KBr discs.

The low resolution mass spectra (LRMS) were recorded: (1) by direct injection of the sample in a Hewlett Packard 5973 MSD spectrophotometer using electron impact (EI) ionization technique with 70 eV ionization energy; or (2) in a Hewlett Packard LCMS 1100 MSD spectrophotometer (an HPLC-coupled quadrupole analyzer) using electrospray chemical ionization technique (API-IS) in positive or negative modes, applying a capillary voltage of 4000 V, a drying temperature of 330°C and using a [1:1] H₂O/MeOH mixture with 1% AcOH as a carrier. The data obtained are expressed in mass units (m/z) and the values in parenthesis correspond to the relative intensities with respect to the base peak (100%). The molecular peak is specified as M⁺.

### Example 1

### Preparation of rac-(4R,5S,6S)-1-benzyloxy-8-bromo-5,6-dihydroxy-5,6-O-(1,1,3,3-tetraisopropyldisiloxane-1,3-diyl)-1-azaspiro[3.5]non-8-en-2,7-dione.

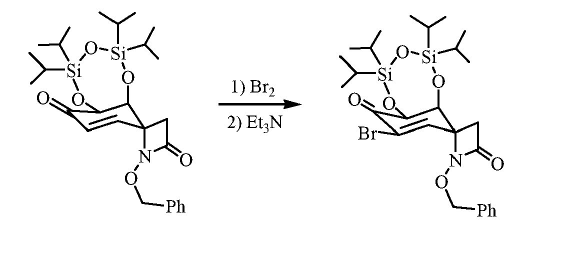

A solution of Br₂ (64.9 mg, 0.40 mmol, 1.08 eq.) in CH₂Cl₂ (1 ml) was added in a solution of *rac*-(4*R*,5*S*,6*S*)-1-benzyloxy-5,6-dihydroxy-5,6-O-(1,1,3,3-tetraisopropyldisiloxane-1,3-diyl)-1-azaspiro[3.5]non-8-ene-2,7-dione (200 mg, 0.376 mmol, 1.0 eq) in CH₂Cl₂ (1 ml) at 0°C. The resulting mixture was stirred at 0°C for 10 minutes. After that time, Et₃N (57.08 mg, 0.564 mmol, 1.5 eq) was added dropwise. The mixture was stirred at room temperature for 10 minutes. The solvent was then removed at reduced pressure. The residue was titrated with Et₂O, it was filtered through Celite and the solvent was removed at reduced pressure. The product was purified by means of column chromatography (hexane/AcOEt; 6:1), *rac*-(4*R*,5*S*,6*S*)-1-benzyloxy-8-bromo-5,6-dihydroxy-5,6-*O*-(1,1,3,3-tetraisopropyldisiloxane-1,3-diyl)-1-azaspiro[3.5]non-8-ene-2,7-dione (210 mg; yld.: 92%) being obtained as a brown oil.
¹H-NMR (δ): 7.37 (5H, m, Ar-H); 6.40 (1H, s); 4.96 (1H, m); 4.84 (1H, d, *J* = 3.0 Hz); 4.41 (1H, d, *J* = 3.0 Hz); 3.21 (1H, d, *J* = 14.2 Hz); 2.61 (1H, d, *J* = 14.2 Hz); 1.04 (28H, m).
LRMS (*API-IS⁺*): *m*/*z* 609 (M-H)⁺, 611 (M+H)⁺, 612 (M+2H)⁺, 628 (M+H₂O)⁺, 629 (M+H₂O+H)⁺.

### Example 2

### Preparation of rac-(4R,5S,6S,8R,9S)-1-benzyloxy-8-bromo-9-cyano-5,6-dihydroxy-5,6-O-(1,1,3,3-tetraisopropyldisiloxane-1,3-diyl)-1-azaspiro[3.5]nonane-2,7-dione

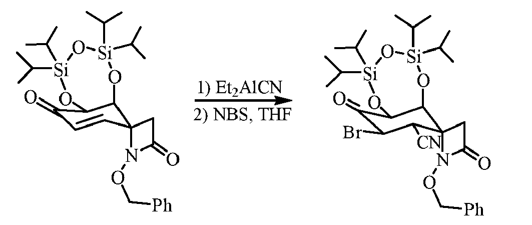

Et₂AlCN (501.48 mg, 4.512 mmol, 1.2 eq.) was added in a solution of *rac*-(4*R*,5*S*,6*S*)-1-benzyloxy-5,6-dihydroxy-5,6-*O-*(1,1,3,3-tetraisopropyldisiloxane-1,3-diyl)-1-azaspiro[3.5]non-8-ene-2,7-dione (2 g, 3.76 mmol, 1.0 eq) in THF (30 ml) at 0°C. The mixture was heated to 80°C for 3 hours. After that time, the mixture was cooled to 0°C and NBS (1.003 g, 5.64 mmol, 1.5 eq.) was added in portions. The resulting solution was stirred a r.t. for 15 minutes. After that time, the solvent was removed at reduced pressure. The residue was titrated with Et₂O, it was filtered through Celite and the solvent was removed at reduced pressure. The product, *rac*-(4*R,*5*S*,6*S*,8*R*,9*S*)-1-benzyloxy-8-bromo-9-cyano-5,6-dihydroxy-5,6-*O*-(1,1,3,3-tetraisopropyldisiloxane-1,3-diyl)-1-azaspiro[3.5]nonane-2,7-dione, was used in the following reaction without purifying (the yield has been considered quantitative).
¹H-NMR (δ): 7.46 (5H, m, Ar-H); 5.30 (1H, d, *J* = 11.1 Hz); 5.19 (1H, d, *J* = 11.1 Hz); 5.07 (1H, dd, *J* = 1.1, 2.3 Hz); 4.34 (1H, d, *J* = 2.3 Hz); 4.22 (1H, dd, *J* = 1.1, 12.6 Hz); 3.88 (1H, d, *J* = 12.6 Hz); 3.06 (1H, d, *J* = 14.5 Hz); 2.85 (1H, d, *J* = 14.5 Hz); 1.25-0.82 (28H, m).
LRMS (*API-IS⁺*): *m*/*z* 581 (M-Br+Na)⁺, 661 (M+Na+H)⁺.

### Example 3

### Preparation of rac-(1R,2S,3S,4R,5S,6R)-5-bromo-1-(2-oxoethyl)-2,3,4-trihydroxy-2,3-O-(1,1,3,3-tetraisopropyldisiloxane-1,3-diyl)-1-(benzyloxyamino)cyclohexane-6-carbonitrile.

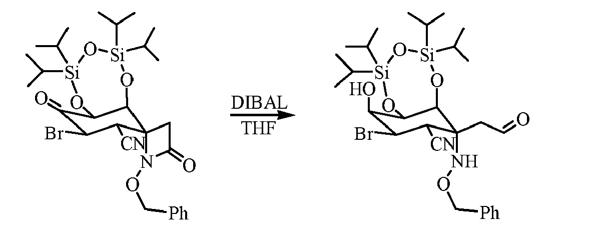

DIBAL (2.169 g, 15.04 mmol, 4 eq.) was added in a solution of *rac-*(4*R*,5*S*,6*S*,8*R*,9*S*)-1-benzyloxy-8-bromo-9-cyano-5,6-dihydroxy-5,6-*O*-(1,1,3,3-tetraisopropyldisiloxane-1,3-diyl)-1-azaspiro[3.5]nonane-2,7-dione (2.39 g, 3.76 mmol, 1 eq.) in THF (50 ml) at 0°C. The mixture was stirred at 0°C for 3 minutes. After that time, AcOEt (30 ml) was added. The resulting mixture was stirred at 0°C for 10 minutes. H₂O (30 ml) was then added. The phases were separated, and the aqueous phase was extracted with AcOEt (4 × 20 ml). The organic phase was dried with anhydrous Na₂SO₄, it was filtered and the solvent was removed at reduced pressure. The product was purified by means of column chromatography (hexane/AcOEt, 9:1), *rac*-(1*R*,2*S*,3*S*,4*R*,5*S*,6*R*)-5-bromo-1-(2-oxoethyl)-2,3,4-trihydroxy-2,3-*O*-(1,1,3,3-tetraisopropyldisiloxane-1,3-diyl)-1-(benzyloxyamino)cyclohexane-6-carbonitrile (866 mg, yld.: 32%) being obtained as a white solid.
Pf.: 103-104°C.
¹H-NMR (δ): 9.74 (1H, s); 7.36 (5H, m, Ar-H); 6.07 (1H, s); 4.77 (1H, d, *J* = 11.7 Hz); 4.68 (1H, d, *J* = 11.7 Hz); 4.49 (1H, m); 4.35 (1H, dd, *J* = 2.5, 11.8 Hz); 4.16 (1H, m); 4.15 (1H, m); 3.54 4 (1H, d, *J* = 11.8 Hz); 3.50 (1H, d, *J* = 18.5 Hz); 2.91 (1H, d, *J* = 4.6 Hz); 2.87 (1H, d, *J* = 18.5 Hz); 1.047 (28H, m).
¹³C-NMR (δ): 199.3, 136.2, 128.8, 128.5, 128.3, 118.3, 76.7, 73.1, 72.5, 71.3, 66.1, 47.6, 43.7, 36.3, 17.6, 17.4, 17.4, 17.2, 17.1, 17.1, 17.0, 17.0, 13.9, 13.4, 13.0, 12.5.
LRMS (*API-IS⁺*): *m*/*z* 641 (M+H)⁺, 1304 (2M+Na+H)⁺.
IR (KBr): ν 3523, 2946, 2867, 2246, 1720, 1464, 1388, 1248, 1162, 1014, 885, 754, 698 cm⁻¹.

### Example 4

### Preparation of rac-(4R,5S,6S,9S)-1-benzyloxy-9-cyano-5,6,7-trihydroxy-5,6-O-(1,1,3,3-tetraisopropyldisiloxane-1,3-diyl)-1-azaspiro[3.5]nonan-2-one.

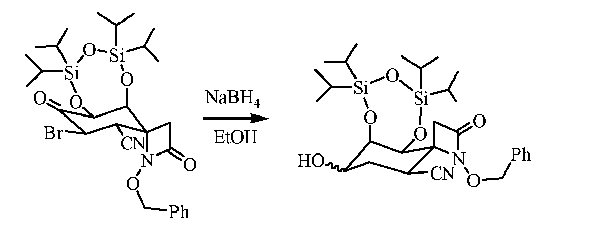

NaBH₄ (15.65 mg, 0.41 mmol, 1.1 eq.) was added in a solution of *rac*-(4*R*,5*S*,6*S*,8*R*,9*S*)-1-benzyloxy-8-bromo-9-cyano-5,6-dihydroxy-5,6-*O*-(1,1,3,3-tetraisopropyldisiloxane-1,3-diyl)-1-azaspiro[3.5]nonane-2,7-dione (239 mg, 0.376 mmol, 1.0 eq) in EtOH (10 ml) at 0°C. The mixture was stirred at 0°C for 30 minutes. After that time, H₂O (20 ml) was added at 0°C. The phases were separated, and the aqueous phase was extracted with AcOEt (3 × 10 ml). The organic phase was dried with anhydrous Na₂SO₄, it was filtered and the solvent was removed at reduced pressure. The product was purified by means of column chromatography (hexane/AcOEt, 9:1), *rac*-(4*R*,5*S*,6*S*,9*S*)-1-benzyloxy-9-cyano-5,6,7-trihydroxy-5,6-*O*-(1,1,3,3-tetraisopropyldisiloxane-1,3-diyl)-1-azaspiro[3.5]nonan-2-one (44 mg, yld.: 21%) being obtained as a white solid.
¹H-NMR (δ): 7.41 (5H, m, Ar-H); 5.04 (1H, d, J = 12.0 Hz); 4.87 (1H, d, J = 12.0 Hz); 4.46 (1H, m); 4.09 (1H, d, *J* = 3.1 Hz); 3.90 (1H, d, *J* = 3.1 Hz); 3.32 (1H, m); 3.10 (1H, d, *J* = 14.1 Hz); 2.66 (1H, d, *J* = 14.1 Hz); 1.69 (2H, m); 1.05 (28H, m).
LRMS (*API-IS⁺*): *m*/*z* 561 (M+H)⁺, 583 (M+Na)⁺, 1144 (2M+Na+H)⁺.

### Example 5

### Preparation of rac-(1R,2S,3S,4R,5S,6R)-5-bromo-1-(2-hydroxyethyl)-2,3,4-trihydroxy-2,3-O-(1,1,3,3-tetraisopropyldisiloxane-1,3-diyl)-1-(benzyloxyamino)cyclohexane-6-carbonitrile.

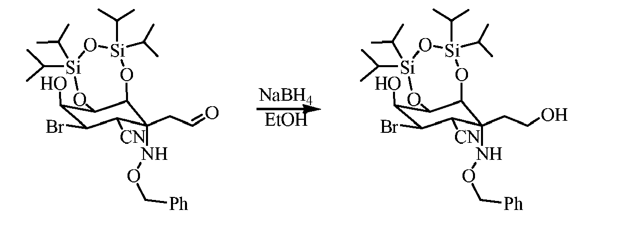

NaBH₄ (26.0 mg, 0.687 mmol, 1.1 eq.) was added in a solution of *rac*-(1*R*,2*S*,3*S*,4*R*,5*S*,6*R*)-5-bromo-1-(2-oxoethyl)-2,3,4-trihydroxy-2,3-*O*-(1,1,3,3-tetraisopropyldisiloxane-1,3-diyl)-1-(benzyloxyamino)-cyclohexane-6-carbonitrile (0.4 g, 0.625 mmol, 1 eq.) in EtOH (16 ml) at 0°C. The resulting mixture was stirred at 0°C for 5 minutes. After that time, an aqueous solution of 0.1 M NaHPO₄ (8 ml) and AcOEt (10 ml) was added. The phases were separated, and the aqueous phase was extracted with AcOEt (3 × 5 ml). The organic phase was dried with anhydrous Na₂SO₄, it was filtered and the solvent was removed at reduced pressure. The product was purified by means of column chromatography (hexane/AcOEt, 8:1), *rac*-(1*R*,2*S*,3*S*,4*R*,5*S*,6*R*)-5-bromo-1-(2-hydroxyethyl)-2,3,4-trihydroxy-2,3-*O*-(1,1,3,3-tetraisopropyldisiloxane-1,3-diyl)-1-(benzyloxyamino)cyclohexane-6-carbonitrile (330 mg, yld.: 82%) being obtained as a colorless oil.
¹H-NMR (δ): 7.36 (5H, m, Ar-H); 5.93 (1H, s); 4.72 (2H, s, -OCH₂Ph); 4.46 (1H, d, *J* = 2.4 Hz); 4.45 (1H, dd, *J* = 2.7, 11.7 Hz); 4.33 (1H, m); 4.19 (1H, m); 3.89 (1H, m); 3.50 (1H, d, *J* = 11.7 Hz); 3.15 (1H, d, *J* = 5.6 Hz); 2.34 (1H, m); 2.26 (1H, m); 2.13 (1H, m); 1.06 (28H, m).

¹³C-NMR (δ): 135.8, 128.7, 128.6, 128.5, 118.6, 77.0, 73.6, 73.4, 71.3, 66.3, 58.1, 49.1, 37.9, 36.3, 17.7, 17.4, 17.4, 17.3, 17.2, 17.2, 17.1, 17.1, 14.1, 13.5, 13.2.
LRMS (*API-IS⁺*): *m*/*z* 643 (M+H)⁺, 644 (M+2H)⁺, 645 (M+3H)⁺, 665 (M+Na+H)⁺, 1308 (2M+Na+H)⁺.
IR (KBr): ν 3468,3027, 2945, 2890, 2867, 2247, 1464, 1386, 1160, 1013, 885, 697 cm⁻¹.

### Example 6

### Preparation of rac-(1R,2S,3S,4R,5S,6R)-5-bromo-1-(2-tert-butyldimethylsilyloxyethyl)-2,3,4-trihydroxy-2,3-O-(1,1,3,3-tetraisopropyldisiloxane-1,3-diyl)-1-(benzyloxyamino)cyclohexane-6-carbonitrile.

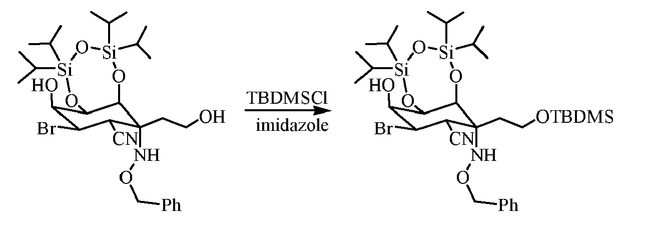

A solution of TBDMSCl (303 mg, 1.697 mmol, 3.3 eq.) in DMF (5 ml) was added in a solution of *rac-*(1*R*,2*S*,3*S*,4*R*,5*S*,6*R*)-5-bromo-1-(2-hydroxyethyl)-2,3,4-trihydroxy-2,3-*O*-(1,1,3,3-tetraisopropyldisiloxane-1,3-diyl)-1-(benzyloxyamino)cyclohexane-6-carbonitrile (330 mg, 0.514 mmol, 1 eq.) and imidazole (115 mg, 1.697 mmol, 3.3 eq.) in DMF (12 ml) at 0°C. The resulting mixture was stirred at room temperature for 16 hours. After that time, H₂O (15 ml) and AcOEt (15 ml) were added. The phases were separated, and the aqueous phase was extracted with AcOEt (4 × 7 ml). The organic phase was washed with a saturated aqueous solution of CuSO₄ (3 × 15 ml) and brine (4 × 7 ml), it was dried with anhydrous Na₂SO₄, it was filtered and the solvent was removed at reduced pressure. The product was purified by means of column chromatography (hexane/AcOEt, 30:1), *rac*-(1*R*,2*S*,3*S*,4*R*,5*S*,6*R*)-5-bromo-1-(2-*tert-butyldimethylsilyloxyethyl*)-2,3,4-trihydroxy-2,3-*O*-(1,1,3,3-tetraisopropyldisiloxane-1,3-diyl)-1-(benzyloxyamino)cyclohexane-6-carbonitrile (264 mg, yld.: 68%) being obtained as a colorless oil.
¹H-NMR (δ): 7.36 (5H, m, Ar-H); 6.00 (1H, s); 4.67 (1H, d, *J* = 15.0 Hz); 4.65 (1H, d, *J* = 15.0 Hz); 4.43 (1H, s_{broad}); 4.37 (2H, m); 4.15 (1H, m); 3.92 (2H, m); 3.52 (1H, d, *J* = 11.9 Hz); 3.21 (1H, d, *J* = 5.6 Hz); 2.32 (1H, dt, *J* = 7.4, 13.6 Hz); 2.04 (1H, dt, *J* = 6.7, 7.4 Hz); 1.06 (28H, m); 0.86 (9H, s); 0.03 (6H, s).
¹³C-NMR (δ): 136.4, 128.6, 128.5, 128.3, 118.7, 77.2, 76.7, 74.3, 73.7, 71.3, 66.1, 59.3, 49.2, 37.5, 37.4, 36.1, 25.8, 18.1, 17.8, 17.4, 17.3, 17.3, 17.2, 17.1, 17.0, 14.2, 13.4, 13.2, 13.1, -5.5.
LRMS (*API-IS⁺*): *m*/*z* 760 (M+2H)⁺, 782 (M+Na+H)⁺, 1540 (2M+Na+H)⁺.

### Example 7

### Reaction of rac-(1R,2S,3S,4R,5S,6R)-5-bromo-1-(2-oxoethyl)-2,3,4-trihydroxy-2,3-O-(1,1,3,3-tetraisopropyldisiloxane-1,3-diyl)-1-(benzyloxyamino)cyclohexane-6-carbonitrile with DBU.

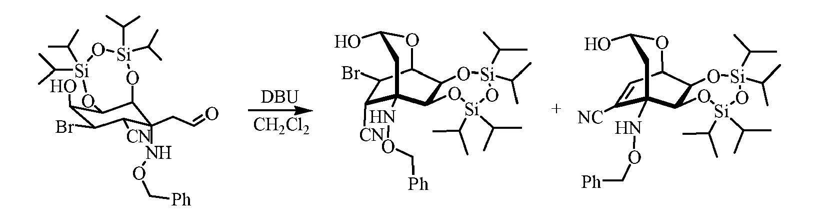

DBU (24 mg, 0.156 mmol, 1 eq.) was added in a solution of *rac*-(1*R*,2*S*,3*S*,4*R*,5*S*,6*R*)-5-bromo-1-(2-oxoethyl)-2,3,4-trihydroxy-2,3-*O*-(1,1,3,3-tetraisopropyldisiloxane-1,3-diyl)-1-(benzyloxyamino)cyclohexane-6-carbonitrile (100 mg, 0.156 mmol, 1 eq.) in CH₂Cl₂ (2 ml) at 0°C. The resulting mixture was stirred at room temperature for 3 hours. After that time, CH₂Cl₂ (10 ml) was added and the mixture was washed with a saturated aqueous solution of CuSO₄ (1 × 5 ml) and brine (1 × 5 ml), it was dried with anhydrous Na₂SO₄, it was filtered and the solvent was removed at reduced pressure. The product was purified by means of column chromatography (hexane/AcOEt, 8:1), rac-(2*S*,3*S*,5*S*,6*R*,8*R*)-1-(benzyloxyamino)-5-bromo-2,3,8-trihydroxy-2,3-*O*-(1,1,3,3-tetraisopropyldisiloxane-1,3-diyl)-9-oxabicyclo[3.2.2]nonane-6-carbonitrile (36 mg, yld.: 36%) being obtained as a colorless oil, and *rac*-(2*S*,3*S*,8*R*)-1-(benzyloxyamino)-2,3,8-trihydroxy-2,3-*O*-(1,1,3,3-tetraisopropyldisiloxane-1,3-diyl)-9-oxabicyclo[3.2.2]non-5-ene-6-carbonitrile (34 mg, yld.: 39%) as a white solid.
*rac*-(2*S*,3*S*,5*S*,6*R*,8*R*)-1-(benzyloxyamino)-5-bromo-2,3,8-trihydroxy-2,3-*O*-(1,1,3,3-tetraisopropyldisiloxane-1,3-diyl)-9-oxabicyclo[3.2.2]nonane-6-carbonitrile. 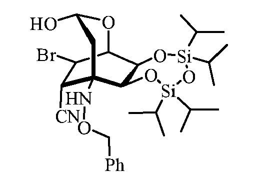 ¹H-NMR (δ): 7.35 (5H, m, Ar-H); 5.87 (1H, s_{broad}); 5.47 (1H, m); 4.76 (1H, d, *J* = 17.3 Hz); 4.72 (1H, d, *J* = 17.3 Hz); 4.61 (1H, dd, *J* = 2.5, 11.7 Hz); 4.39 (2H, m); 4.04 (1H, m); 3.60 (1H, d, *J* = 11.7 Hz); 2.66 (1H, m); 2.31 (1H, dd, *J* = 5.7, 14.1 Hz); 2.03 (1H, m); 1.03 (28H, m).
LRMS (*API-IS⁺*): *m*/*z* 623 (M-H₂O)⁺,624 (M-H₂O+H)⁺, 625 (M-H₂O+2H)⁺, 665 (M+Na+H)⁺.
(2*S*,3*S*,8*R*)-1-(benzyloxyamino)-2,3,8-trihydroxy-2,3-*O-*(1,1,3,3-tetraisopropyldisiloxane-1,3-diyl)-9-oxabicyclo[3.2.2]non-5-ene-6-carbonitrile. 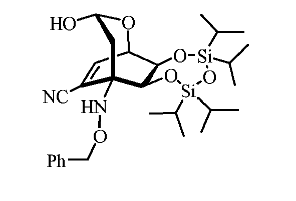 Pf.: 148-149°C.
¹H-NMR (δ): 7.33 (5H, m, Ar-H); 6.53 (1H, dd, *J* = 1.8, 3.6 Hz); 5.87 (1H, s); 5.48 (1H, dd, *J* = 5.3, 9.3 Hz); 4.79 (1H, d, *J* = 11.7 Hz, -OCH₂Ph); 4.73 (1H, d, *J* = 11.7 Hz, -OCH₂Ph); 4.65 (2H, m); 4.43 (1H, dd, *J* = 2.1, 4.9 Hz); 3.68 (1H, d, *J* = 8.0 Hz); 2.47 (1H, dd, *J* = 5.3, 13.3 Hz); 1.81 (1H, d, *J* = 13.3 Hz); 1.06 (28H, m).
¹³C-NMR (δ): 148.6, 136.5, 128.6, 128.4, 128.3, 116.2, 113.5, 99.3, 79.1, 77.4, 73.4, 72.2, 67.4, 42.4, 17.4, 17.4, 17.2, 17.2, 17.0, 17.0, 17.0, 14.0, 13.6, 12.9, 12.4.
LRMS (*API-IS⁺*): *m*/*z* 543 (M-H₂O+H)⁺, 544 (M-H₂O+2H)⁺, 545 (M-H₂O+3H)⁺, 583 (M+Na)⁺.
IR (KBr): ν 3448, 3275, 2946, 2867, 2233, 1639, 1462, 1363, 1246, 1189, 1141, 1070, 1011, 884, 803, 695, 619 cm⁻¹.

### Example 8

### Optimized methods for the preparation of rac-(2S,3S,8R)-1-(benzyloxyamino)-2,3,8-trihydroxy-2,3-O-(1,1,3,3-tetraisopropyldisiloxane-1,3-diyl)-9-oxabicyclo[3.2.2]non-5-ene-6-carbonitrile.

### -Method 1.

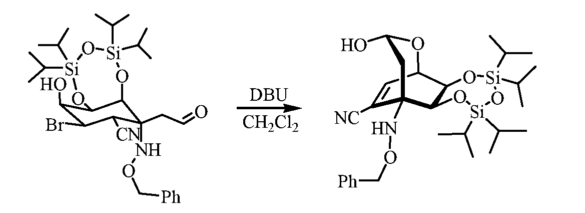

DBU (67 mg, 0.434 mmol, 1.3 eq.) was added in a solution of *rac*-(1*R*,2*S*,3*S*,4*R*,5*S*,6*R*)-5-bromo-1-(2-oxoethyl)-2,3,4-trihydroxy-2,3-*O*-(1,1,3,3-tetraisopropyldisiloxane-1,3-diyl)-1-(benzyloxyamino)cyclohexane-6-carbonitrile (214 mg, 0.333 mmol, 1 eq.) in CH₂Cl₂ (4 ml) at 0°C. The resulting mixture was stirred at room temperature for 16 hours. After that time, CH₂Cl₂ (10 ml) was added and the mixture was washed with a saturated aqueous solution of citric acid (2 × 5 ml), it was dried with anhydrous Na₂SO₄, it was filtered and the solvent was removed at reduced pressure. The product was purified by means of column chromatography (hexane/AcOEt, 8:1), *rac*-(2*S*,3*S*,8*R*)-1-(benzyloxyamino)-2,3,8-trihydroxy-2,3-*O*-(1,1,3,3-tetraisopropyldisiloxane-1,3-diyl)-9-oxabicyclo[3.2.2]non-5-ene-6-carbonitrile (108 mg, yld.: 58%) being obtained as a white solid.

### -Method 2.

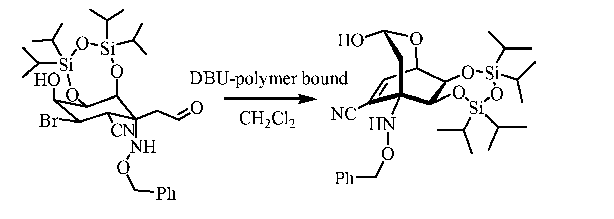

CH₂Cl₂ (11 ml) was added in a mixture of rac-(1*R*,2*S*,3*S*,4*R*,5*S,*6*R*)-5-bromo-1-(2-oxoethyl)-2,3,4-trihydroxy-2,3-*O*-(1,1,3,3-tetraisopropyldisiloxane-1,3-diyl)-1-(benzyloxyamino)cyclohexane-6-carbonitrile (553 mg, 0.862 mmol, 1 eq.) and DBU-polymer bound (1.5g, 1.724 mmol, 2.0 eq.) at 0°C. The resulting mixture was stirred at room temperature for 11 hours. After that time, the mixture was filtered in a büchner with AcOEt and the solvent was removed at reduced pressure. The product was purified by means of column chromatography (hexane/AcOEt, 8:1), *rac*-(2*S*,3*S*,8*R*)-1-(benzyloxyamino)-2,3,8-trihydroxy-2,3-*O*-(1,1,3,3-tetraisopropyldisiloxane-1,3-diyl)-9-oxabicyclo[3.2.2]non-5-ene-6-carbonitrile (256 mg, yld.: 53%) being obtained as a white solid.

### Example 9

### Preparation of rac-(2S,3S,5S,6R,8R)-1-(benzyloxyamino)-5-bromo-2,3,8-trihydroxy-9-oxabicyclo[3.2.2]nonane-6-carbonitrile.

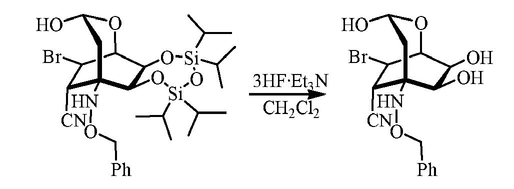

3HF·Et₃N (310 mg, 1.928 mmol, 36 eq.) was added in a solution of *rac*-(2*S*,3*S*,5*S*,6*R*,8*R*)-1-(benzyloxyamino)-5-bromo-2,3,8-trihydroxy-2,3-*O*-(1,1,3,3-tetraisopropyldisiloxane-1,3-diyl)-9-oxabicyclo[3.2.2]nonane-6-carbonitrile (31 mg, 0.054 mmol, 1 eq.) in MeOH (3 ml). The mixture was stirred at room temperature for 24 hours. After that time, an aqueous solution of Na₂HPO₄ (0.1M, 3 ml) and AcOEt (6 ml) was added. The phases were separated, and the aqueous phase was extracted with AcOEt (3 × 5 ml). The organic phase was dried with anhydrous Na₂SO₄, it was filtered and the solvent was removed at reduced pressure. The product was purified by means of column chromatography (hexane/AcOEt, 1:8), *rac*-(2*S*,3*S*,5*S*,6*R*,8*R*)-1-(benzyloxyamino)-5-bromo-2,3,8-trihydroxy-9-oxabicyclo[3.2.2]nonane-6-carbonitrile (8 mg, yld.: 37%) being obtained.
¹H-NMR (δ): 7.34 (5H, m); 5.94 (1H, s_{broad}); 5.56 (1H, m); 4.71 (2H, m); 4.63 (1H, m); 4.49 (1H, m); 4.21 (2H, m); 3.82 (1H, m); 3.53 (1H, m); 3.44 (1H, d, *J* = 11.9 Hz); 3.24 (1H, m); 2.26 (1H, m); 2.04 (1H, m).
LRMS (*API-IS⁺*): *m*/*z* 381 (M-H₂O)⁺, 421 (M+Na)⁺.

### Example 10

### Preparation of rac-(2S,3S,8R)-1-(benzyloxyamino)-2,3,8-trihydroxy-9-oxabicyclo[3.2.2]non-5-ene-6-carbonitrile.

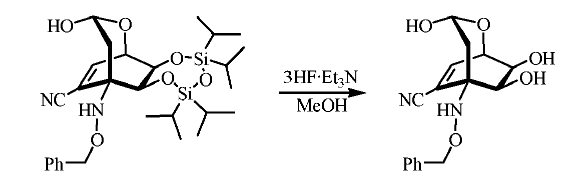

3HF·Et₃N (517 mg, 3.209 mmol, 36 eq.) was added in a solution of *rac*-(2*S*,3*S*,8*R*)-1-(benzyloxyamino)-2,3,8-trihydroxy-2,3-*O*-(1,1,3,3-tetraisopropyldisiloxane-1,3-diyl)-9-oxabicyclo[3.2.2]non-5-ene-6-carbonitrile (50 mg, 0.089 mmol, 1 eq.) in MeOH (5 ml). The mixture was stirred at room temperature for 24 hours. After that time, an aqueous solution of Na₂HPO₄ (0.1M, 5 ml) and AcOEt (10 ml) was added. The phases were separated, and the aqueous phase was extracted with AcOEt (3 × 4 ml). The organic phase was dried with anhydrous Na₂SO₄, it was filtered and the solvent was removed at reduced pressure. The product was purified by means of column chromatography (hexane/AcOEt, 1:8), *rac*-(2*S*,3*S*,8*R*)-1-(benzyloxyamino)-2,3,8-trihydroxy-9-oxabicyclo[3.2.2]non-5-ene-6-carbonitrile (26 mg, yld.: 93%) being obtained.
¹H-NMR (δ): 7.31 (5H, m); 6.64 (1H, s_{broad}); 5.54 (1H, d, *J* = 5.1 Hz); 4.71 (3H, m); 4.33 (1H, m); 4.17 (1H, m); 2.58 (1H, s_{broad}) ; 2.31 (1H, dd, *J* = 5.1, 13.8 Hz); 1.93 (1H, d, *J* = 13.8 Hz);
LRMS (*API-IS⁺*): *m*/*z* 301 (M-H₂O)⁺, 319 (M+H)⁺, 341 (M+Na)⁺.

### Example 11

### Preparation of rac-(2S,3R,4S,8R)-1-(benzyloxyamino)-2,4-dihydroxy-8-methoxy-9-oxabicyclo[3.3.1]non-5-ene-6-carbonitrile.

### -Method 1.

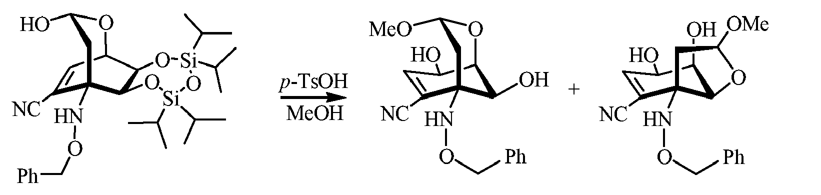

*p*-TsOH (cat.) was added in a solution of *rac*-(2*S*,3*S*,8*R*)-1-(benzyloxyamino)-2,3,8-trihydroxy-2,3-*O*-(1,1,3,3-tetraisopropyldisiloxane-1,3-diyl)-9-oxabicyclo[3.2.2]non-5-ene-6-carbonitrile (102 mg, 0.182 mmol, 1 eq.) in MeOH (7 ml) at room temperature. The resulting mixture was stirred at 55-60°C for 36 hours. After that time, Celite was added and the solvent was removed at reduced pressure. The product was purified by means of column chromatography (hexane/AcOEt, 1:2), *rac-*(2*S*,3*R*,4*S*,8*R*)-1-(benzyloxyamino)-2,4-dihydroxy-8-methoxy-9-oxabicyclo[3.3.1]non-5-ene-6-carbonitrile (51 mg, yld.: 84%) and *rac*-(2*S*,3*R*,4*S*,8*S*)-1-(benzyloxyamino)-2,4-dihydroxy-8-methoxy-9-oxabicyclo[4.3.0]non-5-ene-6-carbonitrile (3 mg, yld.: 5%) both being obtained as a colorless oil.

### -Method 2

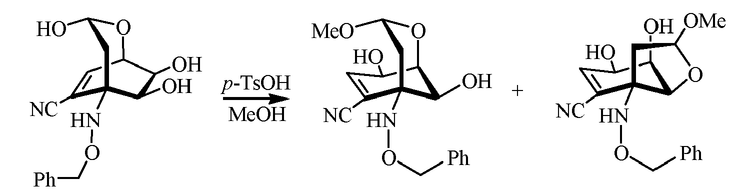

*p*-TsOH (cat.) was added in a solution of *rac*-(2*S*,3*S*,8*R*)-1-(benzyloxyamino)-2,3,8-trihydroxy-9-oxabicyclo[3.2.2]non-5-ene-6-carbonitrile (82 mg, 0.257 mmol, 1 eq.) in MeOH (55 ml) at room temperature. The resulting mixture was stirred at room temperature for 36 hours. After that time, Celite was added and the solvent was removed at reduced pressure. The product was purified by means of column chromatography (hexane/AcOEt, 1:2), *rac*-(2*S*,3*R*,4*S*,8*R*)-1-(benzyloxyamino)-2,4-dihydroxy-8-methoxy-9-oxabicyclo[3.3.1]non-5-ene-6-carbonitrile (70 mg, yld.: 82%) and *rac*-(2*S*,3*R*,4*S*,8*S*)-1-(benzyloxyamino)-2,4-dihydroxy-8-methoxy-9-oxabicyclo[4.3.0]non-5-ene-6-carbonitrile (4 mg, yld.: 5%) both being obtained as a colorless oil.
*rac*-(2*S*,3*R*,4*S*,8*R*)-1-(benzyloxyamino)-2,4-dihydroxy-8-methoxy-9-oxabicyclo[3.3.1]non-5-ene-6-carbonitrile. 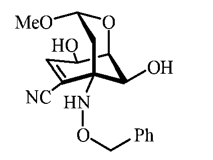 ¹H-NMR (δ): 7.34 (5H, m, Ar-H); 6.66 (1H, dd, *J* = 0.9, 2.7 Hz); 6.23 (1H, s); 5.10 (1H, dd, *J* = 0.7, 5.2 Hz); 4.70 (2H, s); 4.66 (1H, d, *J* = 3.4 Hz); 4.30 (1H, m); 4.18 (1H, m); 3.36 (3H, s); 2.70 (1H, d, *J* = 10.7 Hz); 2.52 (1H, d, *J* = 3.9 Hz); 2.26 (1H, dd, *J* = 5.2, 13.9 Hz); 2.01 (1H, d, *J* = 13.9 Hz).
¹³C-NMR (δ): 146.6, 136.8, 128.6, 128.4, 128.1, 116.7, 116.5, 104.5, 80.1, 77.4, 69.8, 67.1, 67.0, 55.2, 41.8, 29.6, 29.5.
LRMS (*API-IS⁺*): *m*/*z* 301 (M-OMe)⁺, 333 (M+H)⁺, 355 (M+Na)⁺.
IR (KBr): ν 3437,3030,2930, 2225, 1639, 1458, 1365, 1122, 1095, 1034, 924, 753 cm⁻¹.
*rac*-(2*S*,3*R*,4*S*,8*S*)-1-(benzyloxyamino)-2,4-dihydroxy-8-methoxy-9-oxabicyclo[4.3.0]non-5-ene-6-carbonitrile. 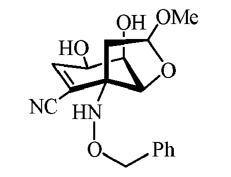 ¹H-NMR (δ): 7.32 (5H, m); 6.66 (1H, dd, *J* = 1.0, 2.6 Hz); 5.16 (1H, t, *J* = 4.9 Hz); 4.72 (2H, m); 4.50 (1H, d, *J* = 2.8 Hz); 4.31 (1H, m); 4.11 (1H, m); 3.91 (1H, m); 3.45 (3H, s); 2.15 (2H, d, *J* = 4.9 Hz).
¹³C-NMR (δ): 147.3, 136.7, 128.5, 128.4, 128.2, 116.4, 115.6, 106.1, 81.9, 77.2, 69.5, 67.7, 67.5, 56.5, 41.8.
LRMS (*API-IS⁺*): *m*/*z* 301 (M-OMe)⁺, 333 (M+H)⁺, 355 (M+Na)⁺.
IR (KBr): ν 3419, 3236, 2925, 2851, 2224, 1454, 1367, 1032, 891, 753 cm⁻¹.

### Example 12

### Preparation of rac-(2S,3R,4S,8R)-1-(benzyloxyamino)-4-tert-butyldimethylsilyloxy-2-hydroxy-8-methoxy-9-oxabicyclo[3.3.1]non-5-ene-6-carbonitrile.

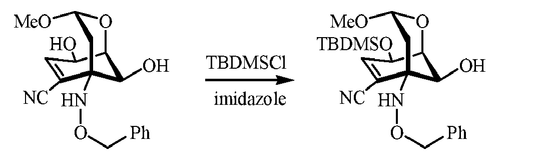

A solution of TBDMSCl (57.15 mg, 0.379 mmol, 3.0 eq.) in DMF (2 ml) was added in a solution of *rac*-(2*S*,3*R*,4*S*,8*R*)-1-(benzyloxyamino)-2,4-dihydroxy-8-methoxy-9-oxabicyclo[3.3.1]non-5-ene-6-carbonitrile (42 mg, 0.126 mmol, 1 eq.) and imidazole (25.8 mg, 0.379 mmol, 3.0 eq.) in DMF (3 ml) at 0°C. The resulting mixture was stirred at room temperature for 16 hours. After that time, H₂O (15 ml) and AcOEt (15 ml) were added. The phases were separated, and the aqueous phase was extracted with AcOEt (4 × 7 ml). The organic phase was washed with a saturated aqueous solution of CuSO₄ (2 × 15 ml) and brine (1 × 7 ml), it was dried with anhydrous Na₂SO₄, it was filtered and the solvent was removed at reduced pressure. The product was purified by means of column chromatography (hexane/AcOEt, 4:1), *rac-*(2*S*,3*R*,4*S*,8*R*)-1-(benzyloxyamino)-4-tert-butyldimethylsilyloxy-2-hydroxy-8-methoxy-9-oxabicyclo[3.3.1]non-5-ene-6-carbonitrile (39 mg, yld.: 70%) being obtained as a colorless oil.
¹H-NMR (δ): 7.31 (5H, m); 6.41 (1H, m); 6.39 (1H, s_{broad}); 5.14 (1H, d, *J* = 5.2 Hz); 4.73 (2H, s); 4.70 (1H, d, *J* = 3.5 Hz); 4.36 (1H, m); 4.05 (1H, m); 3.35 (3H, s); 2.36 (1H, dd, *J* = 5.4, 13.5 Hz); 1.97 (1H, d, *J* = 13.5 Hz); 0.90 (9H, s); 0.11 (3H, s); 0.09 (3H, s).
¹³C-NMR (δ): 147.2, 137.3, 128.8, 128.7, 128.3, 117.1, 116.6, 105.6, 79.3, 77.6, 71.9, 69.2, 67.1, 55.5, 41.4, 25.9, 18.3, -4.6, -4.7.
LRMS (*API-IS⁺*): *m*/*z* 415 (M-OMe)⁺, 469 (M+Na)⁺.
IR (KBr): ν 3487, 3261, 2953, 2929, 2857, 2224, 1725, 1471, 1362, 1255, 1124, 1062, 1044, 895, 839 cm⁻¹.

### Example 13

### Preparation of rac-(2S,3R,4S,8R)-1-(benzyloxyamino)-4-tert-butyldimethylsilyloxy-2-hydroxy-8-methoxy-9-oxabicyclo[3.3.1]non-5-ene-6-carbaldehyde.

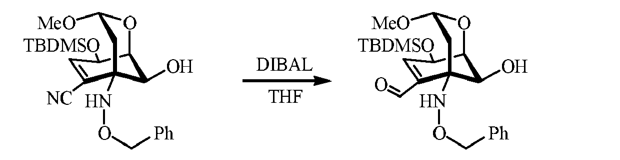

DIBAL (21.3 mg, 0.148 mmol, 2.0 eq.) was added in a solution of *rac*-(2*S*,3*R*,4*S*,8*R*)-1-(benzyloxyamino)-4-tert-butyldimethylsilyloxy-2-hydroxy-8-methoxy-9-oxabicyclo[3.3.1]non-5-ene-6-carbonitrile (33 mg, 0.074 mmol, 1 eq.) in THF (2 ml) at 0°C. The resulting mixture was stirred at 0°C for 2 hours. After that time, AcOEt (2 ml) was added at 0°C, and the mixture was stirred at 0°C for 5 minutes. Saturated NaCl (8 ml) was then added. The phases were separated, and the aqueous phase was extracted with AcOEt (3 × 5 ml). The organic phase was dried with anhydrous Na₂SO₄, it was filtered and the solvent was removed at reduced pressure. The product was purified by means of column chromatography (hexane/AcOEt, 3:1), *rac*-(2*S*,3*R*,4*S*,8*R*)-1-(benzyloxyamino)-4-tert-butyldimethylsilyloxy-2-hydroxy-8-methoxy-9-oxabicyclo[3.3.1]non-5-ene-6-carbaldehyde (8 mg, yld.: 32%) being obtained, and the starting product (8 mg) was recovered.
¹H-NMR (δ): 9.44 (1H, s); 7.34-7.20 (5H, m); 6.51 (1H, dd, *J* = 1.6, 3.3 Hz); 6.44 (1H, s_{broad}); 5.18 (1H, d, *J* = 5.4 Hz); 4.63 (1H, d, *J* = 11.1 Hz); 4.63 (1H, d, *J* = 3.46 Hz); 4.52 (1H, d, *J* = 11.1 Hz); 4.51 (1H, m); 4.09 (1H, m); 3.40 (3H, s); 2.32 (1H, m); 2.27 (1H, d, *J* = 14.1 Hz); 2.16 (1H, dd, *J* = 5.4, 14.1 Hz); 0.92 (9H, s); 0.13 (3H, s); 0.12 (3H, s).
LRMS (*API-IS⁺*) : *m*/*z* 418 (M-OMe)⁺, 450 (M+H)⁺, 473 (M+H+Na)⁺, 880 (2M-H₂O)⁺.

### Example 14

### Preparation of rac-(2S,3S,8R)-1-(benzyloxyamino)-8-(benzoyloxy(methyl)amino)-2,3,8-trihydroxy-2,3-O-(1,1,3,3-tetraisopropyldisiloxane-1,3-diyl)-9-oxabicyclo[3.2.2]non-5-ene-6-carbonitrile

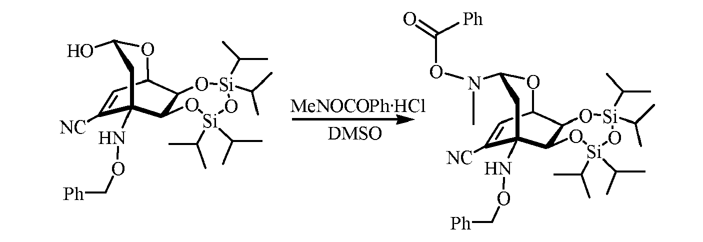

N-methyl-O-benzoylhydroxylamine hydrochloride (46.8 mg, 0.249 mmol, 2 eq.) was added in a solution of *rac-*(2*S*,3*S*,8*R*)-1-(benzyloxyamino)-2,3,8-trihydroxy-2,3-*O*-(1,1,3,3-tetraisopropyldisiloxane-1,3-diyl)-9-oxabicyclo[3.2.2]non-5-ene-6-carbonitrile (70 mg, 0.124 mmol, 1 eq.) in DMSO (1.5 ml). The mixture was stirred at room temperature for 48 hours. After that time, AcOEt (10 ml) was added, it was washed with brine (2 × 3 ml). The organic phase was dried with anhydrous Na₂SO₄, it was filtered and the solvent was removed at reduced pressure. The product was purified by means of column chromatography (hexane/AcOEt, 9:1), *rac*-(2*S*,3*S*,8*R*)-1-(benzyloxyamino)-8-(benzoyloxy(methyl)amino)-2,3,8-trihydroxy-2,3-*O*-(1,1,3,3-tetraisopropyldisiloxane-1,3-diyl)-9-oxabicyclo[3.2.2]non-5-ene-6-carbonitrile (41 mg, yld.: 47%) being obtained as a white solid.
¹H-NMR (δ) : 8.01 (2H, m); 7.57 (1H, m); 7.42 (2H, m); 7.27 (5H, m); 6.64 (1H, S_{broad}) ; 5.50 (1H, dd, *J* = 1.7, 3.4 Hz); 5.06 (1H, m); 4.72 (1H, m); 4.64 (2H, m); 4.49 (1H, m); 2.88 (3H, s); 2.50 (1H, dd, *J* = 7.8, 14.2 Hz); 1.96 (1H, dd, *J* = 2.4, 14.2 Hz); 1.01 (28H, m).
¹³C-NMR (δ): 164.8, 148.1, 137.2, 133.4, 129.6, 128.8, 128.5, 128.3, 127.9, 116.9, 114.9, 96.5, 79.4, 77.2, 74.7, 72.2, 67.3, 41.7, 38.4, 17.5, 17.4, 17.2, 17.1, 17.0, 14.0, 13.6, 13.0, 12.6.
LRMS (*API-IS⁺*) : *m*/*z* 694 (M+H)⁺, 695 (M+2H)⁺, 716 (M+Na)⁺.
IR (KBr): ν 3218, 2945, 2867, 2225, 1747, 1464, 1365, 1257, 1156, 1062, 1010, 886, 755, 707 cm⁻¹.

### Example 15

### Preparation of rac-(2S,3S,8R)-1-(benzyloxyamino)-2,3-dihydroxy-2,3-O-(1,1,3,3-tetraisopropyldisiloxane-1,3-diyl)-7-phenylselenyl-8-methoxy-9-oxabicyclo[3.2.2]non-5-ene-6-carbonitrile.

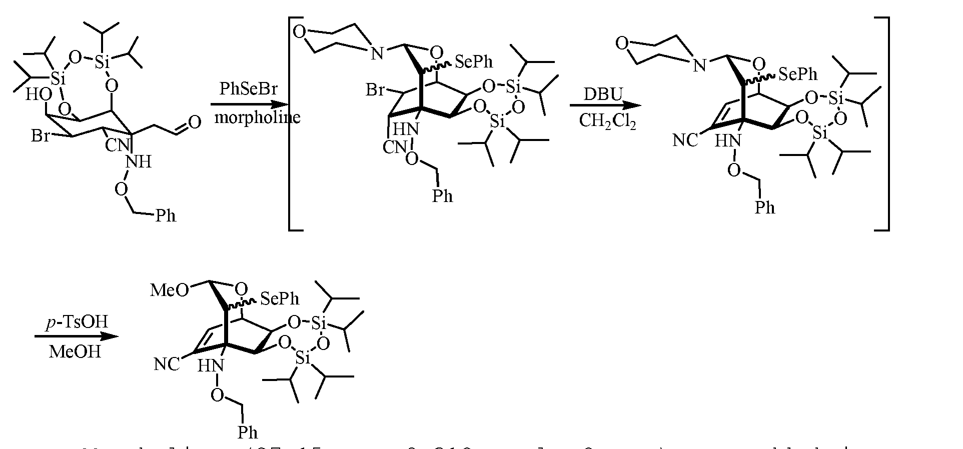

Morpholine (27.15 mg, 0.312 mmol, 2 eq.) was added in a solution of phenylselenium bromide (36.7 mg, 0.156 mmol, 1 eq.) in CH₂Cl₂ (2 ml) at room temperature. The resulting mixture was stirred at room temperature for 15 minutes. A solution of rac-(1*R*,2*S*,3*S*,4*R*,5*S*,6*R*) 5-bromo-1-(2-oxoethyl)-2,3,4-trihydroxy-2,3-*O*-(1,1,3,3-tetraisopropyldisiloxane-1,3-diyl)-1-(benzyloxyamino)cyclohexane-6-carbonitrile (100 mg, 0.156 mmol, 1 eq.) in CH₂Cl₂ (2 ml) was then added. The resulting mixture was stirred at room temperature for 24 hours. A suspension being formed which is filtered through silica gel using CH₂Cl₂ as eluent, and the solvent is removed at reduced pressure. The product is purified by means of column chromatography (hexane/AcOEt, 15:1), *rac*-(2*S*,3*S*,5*S*,6*R*,8*R*)-1-(benzyloxyamino)-5-bromo-2,3-dihydroxy-2,3-*O*-(1,1,3,3-tetraisopropyldisiloxane-1,3-diyl)-7-phenylselenyl-8-morpholin-9-oxabicyclo[3.2.2]nonane-6-carbonitrile (71 mg, yld.: 53%) being obtained as a colorless oil.
DBU (25.11 mg, 0.162 mmol, 1 eq.) was added in a solution of rac- (2*S*, 3*S*,5*S*, 6R, 8R) -1- (benzyloxyamino) -5-bromo-2, 3-dihydroxy-2,3-*O*-(1,1,3,3-tetraisopropyldisiloxane-1,3-diyl)-7-phenylselenyl-8-morpholin-9-oxabicyclo[3.2.2]nonane-6-carbonitrile (141 mg, 0.162 mmol, 1 eq.) in CH₂Cl₂ (4.5 ml) at 0°C. The resulting mixture was stirred at room temperature for 16 hours. After that time, Celite was added and the solvent was removed at reduced pressure. The product was purified by means of column chromatography (hexane/AcOEt, 8:1), *rac-*(2*S*,3*S*,8*R*)-1-(benzyloxyamino)-2,3-dihydroxy-2,3-*O*-(1,1,3,3-tetraisopropyldisiloxane-1,3-diyl)-7-phenylselenyl-8-morpholin-9-oxabicyclo[3.2.2]non-5-ene-6-carbonitrile (82 mg, yld.: 65%) being obtained as an oil.
*p*-TsOH (cat.) was added in a solution of *rac-*(2*S*,3*S*,8*R*)-1-(benzyloxyamino)-2,3-dihydroxy-2,3-*O*-(1,1,3,3-tetraisopropyldisiloxane-1,3-diyl)-7-phenylselenyl-8-morpholin-9-oxabicyclo[3.2.2]non-5-ene-6-carbonitrile (122 mg, 0.155 mmol, 1 eq.) in MeOH (6 ml) at room temperature. The resulting mixture was stirred at room temperature for 36 hours. After that time, Celite was added and the solvent was removed at reduced pressure. The product was purified by means of column chromatography (hexane/AcOEt, 6:1), *rac-*(2*S*,3*S*,8*R*)-1-(benzyloxyamino)-2,3-dihydroxy-2,3-*O*-(1,1,3,3-tetraisopropyldisiloxane-1,3-diyl)-7-phenylselenyl-8-methoxy-9-oxabicyclo[3.2.2]non-5-ene-6-carbonitrile (42 mg, yld.: 37%) being obtained as a brown oil.
¹H-NMR (δ) : 7.54 (10H, m); 5.70 (1H, d, *J* = 7.2 Hz); 5.60 (1H, d, *J* = 6.9 Hz); 5.18 (1H, d, *J* = 7.3 Hz); 5.10 (1H, d, *J* = 2.5 Hz); 5.03 (2H, m); 4.95 (2H, m); 4.70 (1H, m); 4.57 (1H, m); 4.51 (1H, m); 4.46 (1H, m); 4.37 (1H, m); 4.06 (1H, m); 3.98 (1H, m); 3.67 (3H, s); 3.63 (3H, s); 3.56 (3H, s); 3.51 (3H, s); 3.34 (1H, m); 2.35 (1H, m); 1.07 (28H, m).
LRMS (*API-IS⁺*) : *m*/*z* 729 (M-H)⁺, 731 (M+H)⁺, 732 (M+2H)⁺, 733 (M+3H)⁺, 753 (M+Na)⁺.

### Example 16

### Preparation of rac-(4R,5S,6S)-1-benzyloxy-9-cyano-5,6-dihydroxy-5,6-O-(1,1,3,3-tetraisopropyldisiloxane-1,3-diyl)-1-azaspiro[3.5]non-8-ene-2,7-dione (2)

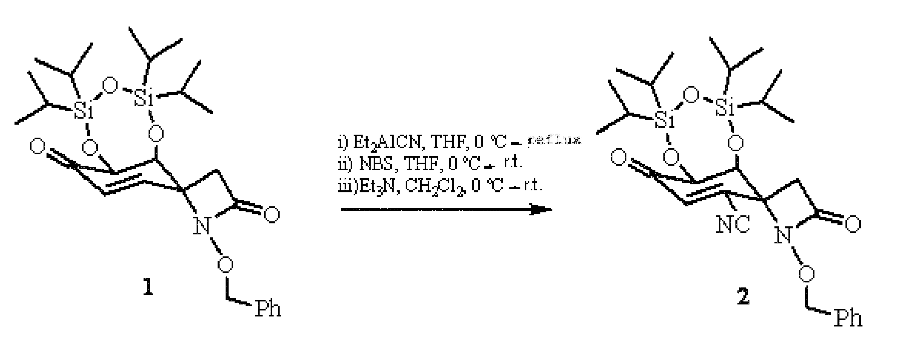

Et₂AlCN (413mg, 3.71 mmol) was added in a solution of 1 (1.65 g, 3.09 mmol) in 32 ml of THF at 0°C. The resulting solution was heated to 80°C for 3 hours. After that time the mixture was cooled to 0°C and NBS (827 mg, 4.64 mmol) was added. The resulting solution was stirred at room temperature for 15 minutes. After that time, the solvent was removed at reduced pressure. The residue was titrated with Et₂O, it was filtered in sieve plate no. 3 through Celite^{®} with Et₂O and the solvent was removed at reduced pressure. The residue was dissolved in 39 ml of CH₂Cl₂. Et₃N (351 mg, 3.47 mmol) was added to the solution at 0°C. The solution was stirred at room temperature for 15 minutes. After that time, Celite^{®} was added to the reaction crude and the solvent was removed at reduced pressure. The product 2 was isolated after column chromatography separation (hexane/AcOEt, 10:1), being obtained as a yellow solid.
P.f.: 115-118 °C.
¹H-NMR: δ 7.37 (5H, m); 6.19 (1H, s); 5.09 (1H, part A *syst. AB, J* = 11.7 Hz); 4.92 (1H, part *B syst. AB, J* = 11.7 Hz); 4.89 (1H, d, *J* = 2.3 Hz); 4.50 (1H, d, *J* = 2.3 Hz); 3.14 (1H, part A *syst. AB, J* = 14.8 Hz); 3.05 (1H, part *B syst. AB, J* = 14.8 Hz); 1.06 (22H, m); 0.94 (3H, d, *J* = 3.5 Hz); 0.90 (3H, d, *J* = 2.0 Hz).
¹³C-NMR: δ 192.0, 164.3, 138.1, 134.7, 129.7, 129.5, 129.1, 127.6, 114.4, 79.9, 77.2, 74.8, 65.2, 42.6, 17.4, 17.3, 17.2, 17.0, 16.8, 16.6, 14.0, 13.5, 13.3, 12.4.
LRMS (*API-IS⁺*) : *m*/*z* 589 (M+H)⁺, 611 (M+Na)⁺, 1200 (2M+Na)⁺.
IR (KBr): ν 3436, 2946, 2890, 2868, 2075, 1799, 1697, 1462, 1372, 1262, 1160, 1105, 1053, 1009, 994, 968, 932, 886, 841, 790, 753, 699 cm⁻¹.
HRMS (ESI⁺) : calculated for C₂₈H₄₁N₂O₆Si₂ (M+H)⁺ 557.2503; 557.2498 found (|Δ| = 1.69).

### Example 17

### Preparation of rac-(4R,5S,6S)-1-benzyloxy-9-cyano-5,6-dihydroxy-7-methylidene-5,6-O-(1,1,3,3-tetraisopropyldisiloxane-1,3-diyl)-1-azaspiro[3.5]non-8-en-2-one (3)

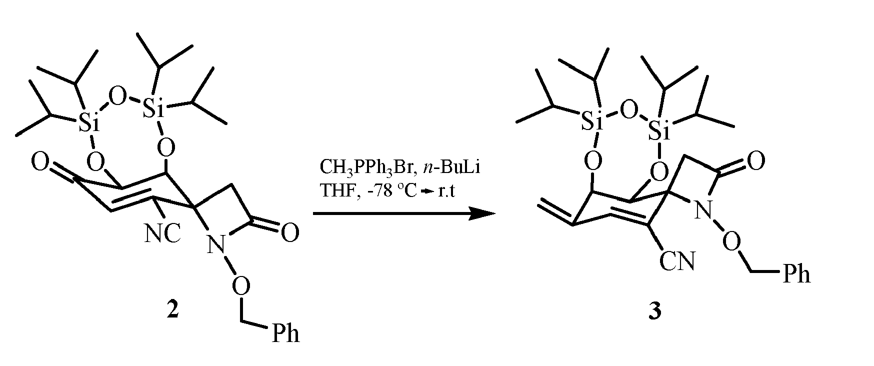

n-BuLi (93 mg, 1.45 mmol) was added dropwise in a suspension of triphenylphosphonium bromide (499 mg, 1.40 mmol) in 20 ml of THF at -78°C. The suspension was stirred at -78°C for 15 minutes. After that time, 2 (622 mg, 1.12 mmol) dissolved in 13 ml of THF was added dropwise at -78°C. The suspension was stirred at -78°C for 1 hour and then at 0°C for 18 hours. After that time, the solvent was removed at reduced pressure. The residue was titrated with Et₂O (the process is repeated 3 times). Celite^{®} was added to the reaction crude and the solvent was removed at reduced pressure. The product 3 was filtered on *60 Merck* silica with Et₂O a brown solid being obtained (when product 3 was isolated after column chromatography separation (hexane/AcOEt, 20:1) it was obtained as a white solid). The reaction crude was used in the dihydroxylation reaction of the exocyclic double bond.
¹H-NMR: δ 7.36 (5H, m); 6.74 (1H, s); 5.63 (1H, s); 5.50 (1H, m); 5.07 (1H, part A *syst. AB, J* = 11.4 Hz); 4.94 (1H, part *B syst. AB, J* = 11.4 Hz); 4.86 (1H, dt, *J* = 2.6, 1.4 Hz); 4.36 (1H, d, *J* = 2.6 Hz); 3.22 (1H, part *A syst. AB, J* = 14.3 Hz); 2.83 (1H, part *B syst. AB, J*= 14.3 Hz); 1.07 (28H, m).
¹³C-NMR: δ 64.8, 143.7, 140.4, 135.2, 129.1, 128.8, 128.7, 125.2, 116.4, 79.3, 72.4, 65.1, 41.0, 17.5, 17.4, 17.3, 17.2, 17.0, 16.9, 14.0, 13.7, 13.4, 13.0.
LRMS (*API-IS⁺*) : *m*/*z* 513 (M-ⁱPr+H)⁺, 555 (M+H)⁺, 577 (M+Na)⁺, 1131 (2M+Na)⁺.
IR (film): ν 3436, 3033, 2945, 2890, 2868, 2220, 1787, 1770, 1629, 1464, 1385, 1250, 1156, 1060, 1013, 967, 912, 886, 843, 792, 747, 698 cm⁻¹.

### Example 18

### Preparation of rac-(4R,5S,6S,7R)-1-benzyloxy-9-cyano-5,6,7-trihydroxy-7-(hydroxymethyl)-5,6-O-(1,1,3,3-tetraisopropyldisiloxane-1,3-diyl)-1-azaspiro[3.5]non-8-en-2-one (4)

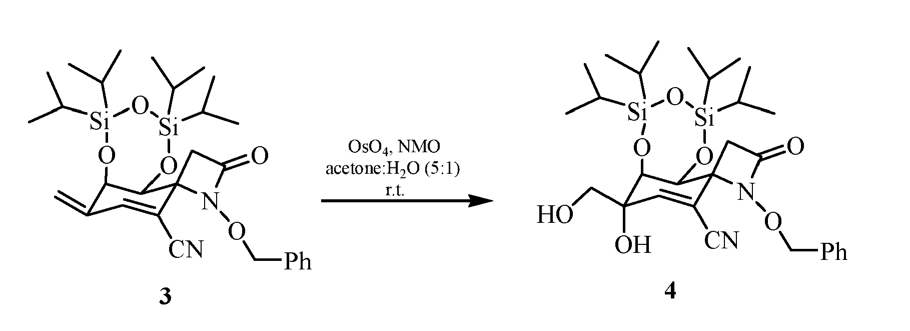

NMO (290 mg, 2.47 mmol) and dissolved OsO₄ (0.85 ml, 2.5% by weight in *^{t}*BuOH, 0.07 mmol) were added in a solution of 3 (624 mg, 1.125 mmol) in 16 ml of acetone and 3.2 ml of H₂O at room temperature. The solution was stirred at room temperature for 17 hours. After that time, Na₂S₂O₃ (10%, 3.4 ml) was added at room temperature and it was stirred for 5 minutes. The phases were separated in an extraction funnel. The aqueous phase was extracted with AcOEt (7 x 15 ml). The combined organic extracts were dried with anhydrous Na₂SO₄, they were filtered and the solvent was removed at reduced pressure. Celite^{®} and AcOEt were added to the reaction crude and the solvent was removed at reduced pressure. Product 4 was filtered on 60 *Merck* silica (hexane/AcOEt, 1:1) a brown oil being obtained (when product 4 was isolated after column chromatography separation (hexane/AcOEt, 3:1) it was obtained as a white solid). The reaction crude was used in the protection reaction of the generated diol.
P.f.: 128-131 °C.
¹H-NMR : δ 7.45 (2H, m); 7.37 (3H, m); 6.38 (1H, d, *J* = 1.4 Hz); 5.11 (1H, part A *syst. AB, J* = 12.1 Hz), 4.96 (1H, part *B syst. AB, J* = 12.1 Hz); 4.65 (1H, d, *J* = 2.4 Hz); 4.31 (1H, dd, *J* = 2.4, 1.4 Hz); 3.68 (1H, part A *syst. AB, J* = 10.9 Hz); 3.63 (1H, part *B syst. AB, J* = 10.9 Hz), 3.24 (1H, part A *syst. AB, J* = 14.2 Hz); 2.65 (1H, part *B syst. AB, J* = 14.2 Hz), 2.26 (1H, S_{broad}, OH); 2.04 (1H, S_{broad}, OH); 1.05 (28H, m).
¹³C-NMR: δ 165.0, 143.2, 136.0, 129.3, 128.8, 128.6, 118.6, 115.4, 79.0, 74.9, 72.2, 67.8, 65.4, 64.9, 40.2, 17.6, 17.5, 17.1, 17.0, 16.9, 14.4, 14.0, 13.3, 13.2.
LRMS (*API-IS⁺*) : *m*/*z* 571 (M-H₂O+H)⁺, 589 (M+H)⁺, 611 (M+Na)⁺, 1199 (2M+Na)⁺.
IR (KBr): ν 3438, 2947, 2869, 2231, 1778, 1633, 1466, 1389, 1369, 1249, 1214, 1153, 1094, 1006, 971, 929, 885, 840, 790, 745, 697 cm⁻¹.
HRMS (ESI⁺) : calculated for C₂₉H₄₈N₃O₇Si₂ (M+NH₄)⁺ 606.3031; 606.3040 found (|Δ| = 2.41).

### Example 19

### Preparation of rac-(4R,5S,6S,7R)-1-benzyloxy-9-cyano-5,6,7-trihydroxy-7-(hydroxymethyl)-7,10-O-(propane-2,2-diyl)-5,6-O-(1,1,3,3-tetraisopropyldisiloxane-1,3-diyl)-1-azaspiro[3.5]non-8-en-2-one (5)

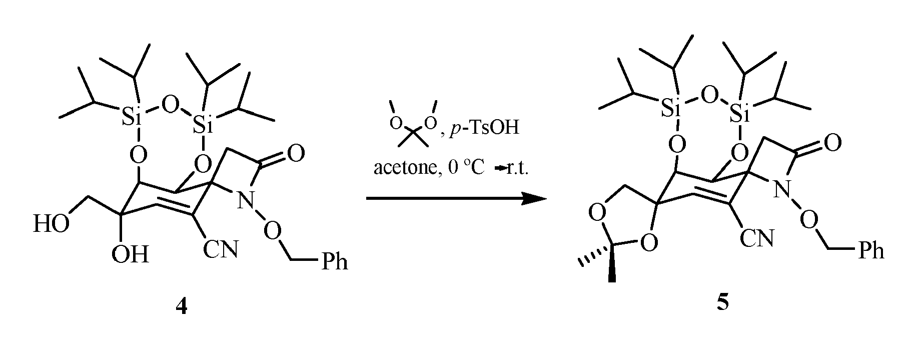

2,2-dimethoxypropane (310 mg, 2.98 mmol) and a catalytic amount of p-toluenesulfonic acid were added in a solution of 4 (585 mg, 0.99 mmol) in 40 ml of acetone at 0°C. The solution was stirred at room temperature for 18 hours. After that time, Celite^{®} was added to the reaction crude and the solvent was removed at reduced pressure. The product 5 was isolated after column chromatography separation (hexane/AcOEt, 6:1), being obtained as a white solid.
P.f.: 159-162 °C.
¹H-NMR: δ 7.41 (3H, m); 7.35 (2H, m); 6.31 (1H, d, *J* = 1.4 Hz) ; 5.08 (1H, part A *syst. AB, J* = 11.5 Hz); 5.01 (1H, part *B syst. AB, J* = 11.5 Hz); 4.68 (1H, d, *J* = 2.3 Hz); 4.33 (1H, dd, *J* = 2.3, 1.4 Hz); 4.25 (1H, part A *syst. AB, J* = 9.7 Hz); 3.84 (1H, part *B syst. AB, J* = 9.7 Hz); 3.21 (1H, part A *syst. AB, J* = 14.0 Hz); 2.64 (1H, part *B syst. AB, J* = 14.0 Hz); 1.44 (3H, s); 1.43 (3H, s); 1.06 (28H, m).
¹³C-NMR: δ 164.6, 144.5, 135.3, 128.8, 128.6, 128.5, 117.9, 115.2, 111.5, 79.3, 79.0, 74.8, 70.6, 68.5, 64.3, 40.4, 26.9, 26.4, 17.7, 17.6, 17.5, 17.1, 17.0, 16.9, 14.3, 14.0, 13.3.
LRMS (*API-IS⁺*) : *m*/*z* 629 (M+H)⁺, 651 (M+Na)⁺, 674 (M+2Na)⁺, 1279 (2M+Na)⁺.
IR (KBr): ν 3436, 3063, 2947, 2896, 2869, 2225, 1785, 1641, 1466, 1373, 1259, 1219, 1182, 1151, 1125, 1097, 1064, 999, 946, 926, 886, 845, 798, 759, 745, 697, 663, 607, 517 cm⁻¹.
HRMS (ESI⁺) : calculated for C₃₂H₄₉N₂O₇Si₂ (M+H)⁺ 629.3078; 629.3100 found (|Δ| = 1.66).

### Example 20

### Preparation of rac-(1R,2S,3S,4R)-1-(benzyloxyamino)-2,3,4-trihydroxy-4-(hydroxymethyl)-4,7-O-(propane-2,2-diyl)-2,3-O-(1,1,3,3-tetraisopropyldisiloxane-1,3-diyl)-1-(2-oxoethyl)cyclohex-5-ene-6-carbonitrile (6)

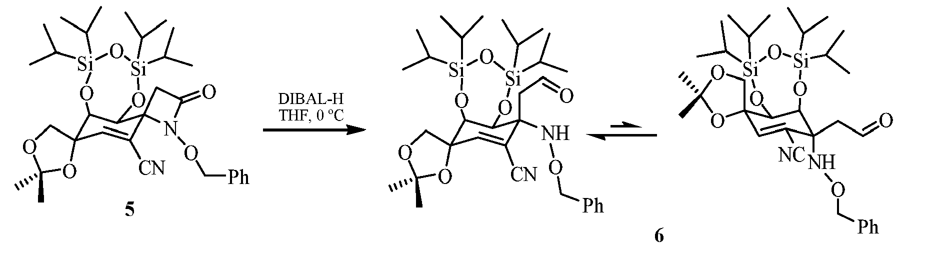

DIBAL-H (318 mg, 2.20 mmol) was added in a solution of 5 (462 mg, 0.74 mmol) in 26 ml of THF at 0°C. The solution was stirred at 0°C for 1 hour. After that time, 26 ml of AcOEt was added at 0°C and it was stirred at 0°C for 5 minutes. Saturated NaCI (15 ml) was then added at 0°C. The phases were separated in an extraction funnel. The aqueous phase was extracted with AcOEt (6 x 15 ml). The combined organic extracts were dried with anhydrous Na₂SO₄, they were filtered and the solvent was removed at reduced pressure. Celite^{®} and AcOEt were added to the reaction crude and the solvent was removed at reduced pressure. The product 6 was isolated after column chromatography separation (hexane/AcOEt, 10:1), being obtained as a colorless oil.
¹H-NMR: δ 9.82 (1H, t, *J* = 2.5 Hz); 7.33 (5H, m); 6.59 (1H, s); 5.84 (1H, S_{broad}, NH); 4.84 (1H, part A *syst. AB, J* = 11.9 Hz); 4.79 (1H, *part B syst. AB, J* = 11.9 Hz); 4.75 (1H, d, *J* = 2.9 Hz); 4.53 (1H, d, *J* = 2.9 Hz); 4.36 (1H, part A *syst. AB, J* = 9.4 Hz); 3.85 (1H, part *B syst. AB, J* = 9.4 Hz); 2.84 (1H, dd, *J* = 16.8, 2.5 Hz); 2.74 (1H, dd, *J* = 16.8, 2.5 Hz); 1.47 (3H, s); 1.43 (3H, s); 1.07 (28H, m).
¹³C-NMR: δ 198.8, 147.3, 136.9, 128.4, 128.2, 128.0, 116.9, 116.7, 110.7, 80.3, 76.8, 74.0, 71.1, 69.6, 64.0, 46.3, 27.3, 26.3, 17.8, 17.6, 17.5, 17.4, 17.1, 17.0, 13.9, 13.8, 13.7.
LRMS (*API-IS⁺*): *m*/*z* 631 (M+H)⁺, 653 (M+Na)⁺, 1283 (2M+Na)⁺.
IR (film): ν 3421, 2947, 2890, 2869, 2226, 1722, 1465, 1383, 1372, 1256, 1217, 1116, 1060, 1008, 886, 853, 757, 698 cm⁻¹.

### Example 21

### Preparation of rac-(1R,2S,3S,4R,8R)-1-(benzyloxyamino)-2,4,8-trihydroxy-4-(hydroxymethyl)-4,10-O-(propane-2,2-diyl)-2,8-O-(1,1,3,3-tetraisopropyldisiloxane-1,3-diyl)-9-oxabicyclo[3.3.1]non-5-ene-6-carbonitrile (7)

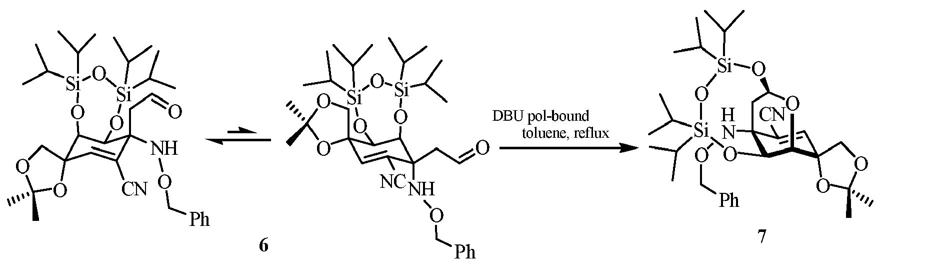

27 ml of toluene was added in a mixture of 6 (469 mg, 0.74 mmol) and bound DBU (969 mg 1.15 mmol/g load, 1.12 mmol) at room temperature. The suspension was heated to 120°C for 18 hours. After that time, the suspension was filtered in Büchner funnel under vacuum. The solid was washed alternatively with CH₂Cl₂ and MeOH. Then the solvent was removed at reduced pressure. Celite^{®} and AcOEt were added to the reaction crude and the solvent was removed at reduced pressure. Product 7 was isolated after column chromatography separation (hexane/AcOEt, 20:1), being obtained as a colorless oil.
¹H-NMR : δ 7.34 (5H, m); 6.55 (1H, s); 5.61 (1H, d, *J* = 5.0 Hz); 5.51 (1H, S_{broad}, NH); 4.82 (1H, part A *syst. AB, J* = 2.9 Hz); 4.81 (1H, part *B syst. AB, J* = 2.9 Hz); 4.72 (2H, s); 4.53 (1H, part A *syst. AB, J* = 9.4 Hz); 3.69 (1H, part *B syst. AB, J* = 9.4 Hz); 2.38 (1H, dd, *J* = 14.4, 5.0 Hz); 1.84 (1H, dd, *J* = 14.4, 0.7 Hz); 1.50 (3H, s); 1.44 (3H, s); 1.07 (28H, m).
¹³C-NMR: δ 150.5, 136.9, 128.8, 128.4, 128.0, 116.5, 114.6, 109.9, 96.3, 81.2, 80.4, 77.0, 70.7, 69.1, 68.1, 45.2, 27.5, 26.0, 17.8, 17.7, 17.5, 17.3, 17.2, 17.1, 17.0, 16.9, 14.2, 13.7, 13.2, 12.7.
LRMS (*API-IS⁺*) : *m*/*z* 631 (M+H)⁺, 653 (M+Na)⁺.
IR (film): ν 3249, 2946, 2868, 2225, 1630, 1497, 1464, 1382, 1370, 1320, 1306, 1287, 1254, 1215, 1142, 1105, 1080, 1050, 992, 961, 887, 866, 833, 756, 698 cm⁻¹.
HRMS (ESI⁺) : calculated for C₃₂H₅₁N₂O₇Si₂ (M+H)⁺ 631.3235; 631.3229 found (|Δ| = 1.39).

### Example 22

### Preparation of rac-(1R,2S,3S,4R,8R)-1-(benzyloxyamino)-2,4,8-trihydroxy-4-(hydroxymethyl)-4,10-O-(propane-2,2-diyl)-2,8-O-(1,1,3,3-tetraisopropyldisiloxane-1,3-diyl)-9-oxabicyclo[3.3.1]non-5-ene-6-carbaldehyde (8)

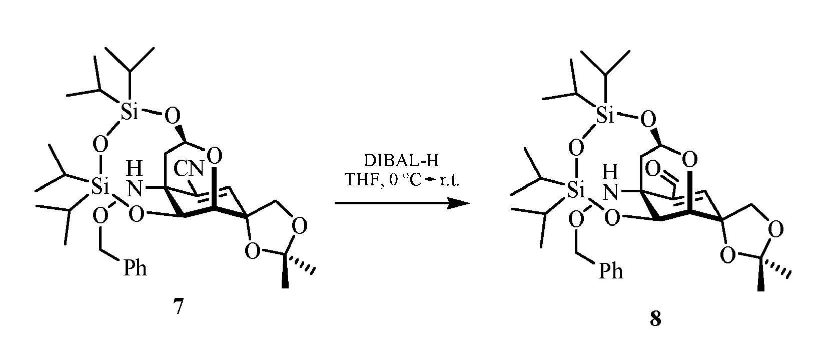

DIBAL-H (169 mg, 1.17 mmol) was added in a solution of 7 (185 mg, 0.29 mmol) in 15 ml of THF at 0°C. The solution was stirred at 0°C for 2 hours and at room temperature for 2 hours. After that time 15 ml of AcOEt was added at 0°C and it was stirred at 0°C for 5 minutes. Saturated NaCI (8 ml) was then added. The phases were separated in an extraction funnel. The aqueous phase was extracted with AcOEt (5 x 10 ml). The combined organic extracts were dried with anhydrous Na₂SO₄, they were filtered and the solvent was removed at reduced pressure. Celite^{®} and AcOEt were added to the reaction crude and the solvent was removed at reduced pressure. Product 8 was isolated after column chromatography separation (hexane/AcOEt, 15:1), being obtained as a colorless oil.
¹H-NMR : δ 9.36 (1H, s); 7.21 (5H, m); 6.58 (1H, s); 6.15 (1H, s, NH); 5.48 (1H, d, *J* = 5.0 Hz); 4.78 (1H, part A *syst. AB, J* = 2.8 Hz); 4.73 (1H, *part* B *syst. AB, J* = 2.8 Hz); 4.65 (1H, part A *syst. AB, J* = 12.1 Hz); 4.56 (1H, part A *syst. AB, J* = 9.3 Hz); 4.53 (1H, part *B syst. AB, J* = 12.1 Hz); 3.67 (1H, *part B syst. AB, J* = 9.3 Hz); 2.21 (1H, dd, *J* = 14.2, 5.0 Hz); 2.00 (1H, d, *J* = 14.2 Hz); 1.47 (3H, s), 1.42 (3H, s); 1.03 (28H, m).
¹³C-NMR: δ 193.9, 156.2, 137.7, 137.3, 128.4, 127.9, 127.8, 109.8, 96.8, 82.0, 81.4, 76.1, 71.3, 69.4, 66.4, 44.9, 27.7, 26.1, 17.9, 17.8, 17.6, 17.4, 17.3, 17.0, 16.8, 14.6, 13.8, 13.2, 12.7.
LRMS (*API-IS⁺*) : *m*/*z* 634 (M+H)⁺, 656 (M+Na)⁺.
IR (film): ν 3436, 2945, 2868, 1677, 1634, 1464, 1370, 1255, 1205, 1130, 1090, 1052, 1004, 920, 885, 838, 747, 701, 605 cm⁻¹.
HRMS (ESI⁺) : calculated for C₃₂H₅₂NO₈Si₂ (M+H)⁺ 634.3231; 634.3246 found (|Δ| = 3.04).

### Example 23

### Preparation of rac-(1R,2S,3S,4R,8R)-1-(benzyloxyamino)-2,4,8-trihydroxy-4,6-bis-(hydroxymethyl)-4,10-O-(propane-2,2-diyl)-2,8-O-(1,1,3,3-tetraisopropyldisiloxane-1,3-diyl)-9-oxabicyclo[3.3.1]non-5-ene (9)

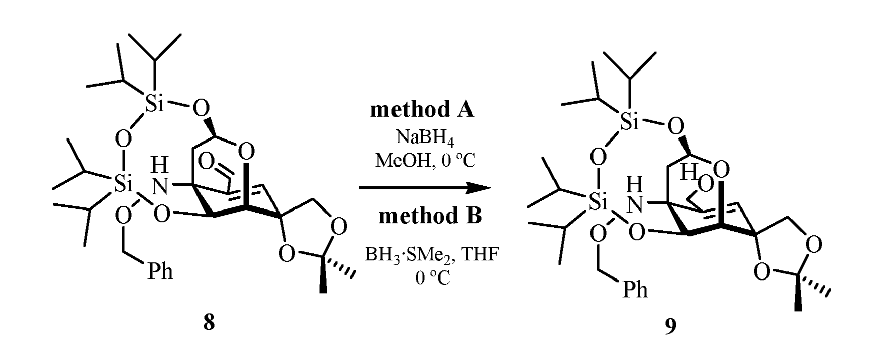

### Method A

NaBH₄ (9 mg, 0.25 mmol) was added in a suspension of 8 (141 mg, 0.22 mmol) in 3.3 ml of MeOH at 0°C. The solution was stirred at 0°C for 45 minutes. After that time, Na₂HPO₄ (0.1M, 11ml) was added at 0°C. The phases were separated in an extraction funnel. The aqueous phase was extracted with AcOEt (6 x 4 ml). The combined organic extracts were dried with anhydrous Na₂SO₄, they were filtered and the solvent was removed at reduced pressure. Celite^{®} and AcOEt were added to the reaction crude and the solvent was removed at reduced pressure. Product 9 was isolated after column chromatography separation (hexane/AcOEt, 4:1), being obtained as a white solid.

### Method B

BH₃(CH₃)₂S (29 mg, 0.39 mmol) was added in a solution of 8 (49 mg, 0.08 mmol) in 2 ml of THF at 0°C. The solution was stirred at 0°C for 15 minutes. After that time, H₂O₂ (33%, 1ml) and saturated NaHCO₃ (1 ml) were added at 0°C and it was stirred at 0°C for 5 minutes. The phases were separated in an extraction funnel. The aqueous phase was extracted with AcOEt (4 x 2 ml). The combined organic extracts were dried with anhydrous Na₂SO₄, they were filtered and the solvent was removed at reduced pressure. Celite^{®} and AcOEt were added to the reaction crude and the solvent was removed at reduced pressure. Product 9 was isolated after column chromatography separation (hexane/AcOEt, 4:1), being obtained as a white solid.
¹H-NMR: δ 7.31 (5H, m); 5.75 (1H, t, *J* = 1.1 Hz); 5.59 (1H, S_{broad}, NH); 5.57 (1H, d, *J* = 5.1 Hz); 4.84 (1H, d, *J* = 3.0 Hz); 4.72 (1H, d, *J* = 3.0 Hz); 4.71 (1H, part A *syst. AB, J* = 13.1 Hz); 4.67 (1H, part *B syst. AB, J* = 13.1 Hz); 4.50 (1H, part A *syst. AB, J* = 8.9 Hz); 4.25 (1H, dd, *J* = 13.2, 1.1 Hz); 4.07 (1H, d, *J* = 13.2 Hz); 3.60 (1H, part *B syst. AB, J* = 8.9 Hz); 2.73 (1H, S_{broad}, OH); 2.36 (1H, dd, *J* = 14.1, 5.1 Hz) ; 1.88 (1H, d, *J* = 14.1Hz); 1.48 (3H, s); 1.44 (3H, s); 1.09 (20H, m); 1.00 (8H, m).
¹³C-NMR: δ 137.3, 134.9, 134.2, 128.7, 128.6, 128.3, 109.1, 96.8, 82.8, 80.7, 76.8, 71.9, 69.8, 69.2, 63.9, 43.8, 27.8, 26.5, 18.1, 18.0, 17.8, 17.6, 17.5, 17.4, 17.3, 17.1, 14.5, 14.0, 13.5, 12.9.
LRMS (*API-IS⁺*) : *m*/*z* 636 (M+H)⁺, 658 (M+Na)⁺.
IR (film): ν 3415, 3060, 3027, 2945, 2896, 2868, 1651, 1497, 1464, 1368, 1253, 1213, 1121, 1090, 1048, 992, 885, 834, 747, 698 cm⁻¹.
HRMS (ESI⁺) : calculated for C₃₂H₅₄NO₈Si₂ (M+H)⁺ 636.3388; 636.3375 found ( |Δ| = 1.01).

### Example 24

### Preparation of rac-(1R,2S,3S,4R,8R)-1-(benzyloxyamino)-6-[(phenylselanyl)methyl]-2,4,8-trihydroxy-4-(hydroxymethyl)-4,10-O-(propane-2,2-diyl)-2,8-O-(1,1,3,3-tetraisopropyldisiloxane-1,3-diyl)-9-oxabicyclo[3.3.1]non-5-ene (10)

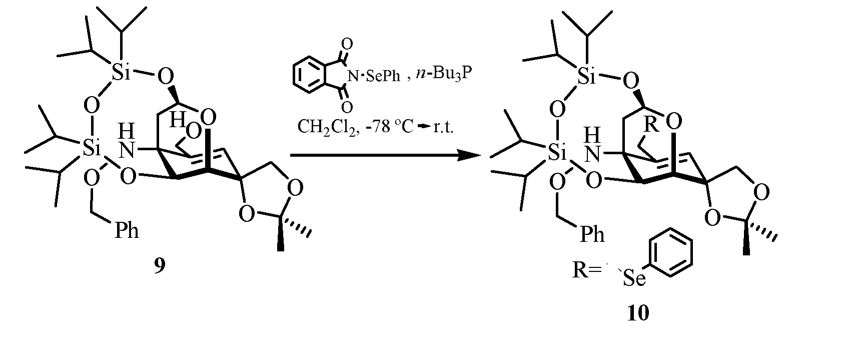

N-(phenylselanyl)phthalimide (38 mg, 0.13 mmol) and n-Bu₃P (27 mg, 0.13 mmol) were added in a solution of 9 (61 mg, 0.10 mmol) in 1 ml of CH₂Cl₂ at -78°C. The suspension was stirred at 0°C for 2 hours and at room temperature for 2 hours. N-(phenylselanyl) phthalimide (74 mg, 0.25 mmol) and *n*-Bu₃P (54 mg, 0.26 mmol) were then added at 0°C. The suspension was stirred at room temperature for 1 hour. After that time, the suspension is filtered in sieve plate no. 3 under vacuum with CH₂Cl₂, and the solvent was removed at reduced pressure. Celite^{®} and AcOEt were added to the reaction crude and the solvent was removed at reduced pressure. Product 10 was isolated after column chromatography separation (hexane/AcOEt, 20:1), being obtained as a white solid.
¹H-NMR: δ 7.50 (2H, m); 7.31 (8H, m); 5.56 (1H, s); 5.52 (1H, d, J= 5.2 Hz); 5.42 (1H, S_{broad}, NH); 4.76 (1H, part A *syst. AB, J* = 2.9 Hz); 4.67 (1H, part *B syst. AB, J* = 2.9 Hz); 4.66 (1H, part A *syst. AB, J* = 12.0 Hz); 4.60 (1H, part *B syst. AB, J* = 12.0 Hz); 4.44 (1H, part A *syst. AB, J* = 8.9 Hz); 3.54 (1H, part A *syst. AB, J* = 12.2 Hz); 3.48 (1H, part *B syst. AB, J* = 12.2 Hz); 3.44 (1H, part *B syst. AB, J* = 8.9 Hz); 2.62 (1H, dd, *J* = 14.0, 5.2 Hz); 1.72 (1H, d, *J* = 14.0 Hz); 1.41 (3H, s); 1.36 (3H, s), 1.05 (28H, m).
LRMS (*API-IS⁺*) : *m*/*z* 776 (M+H)⁺, 798 (M+Na)⁺, 1576 (2M+Na+3H)⁺.

### Example 25

### Preparation of rac-(1S,2S,3S,4R,8R)-1-(benzyloxyamino)-2,4,8-trihydroxy-4-(hydroxymethyl)-6-{[(2-nitrophenyl)selanyl]methyl}-4,10-O-(propane-2,2-diyl)-2,8-O-(1,1,3,3-tetraisopropyldisiloxane-1,3-diyl)-9-oxabicyclo[3.3.1]non-5-ene (11)

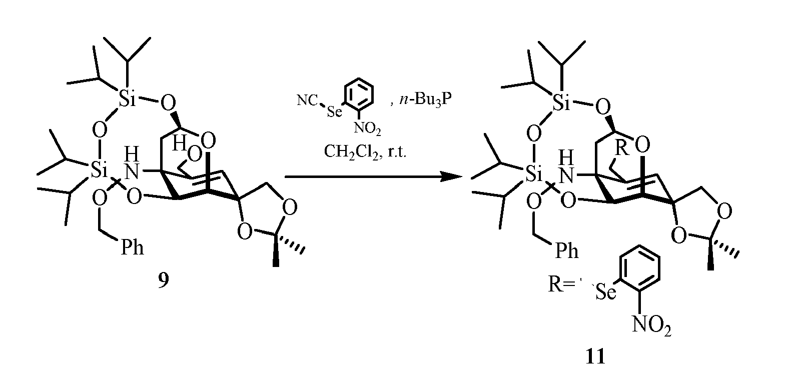

2-nitrophenyl selenocyanate (36 mg, 0.16 mmol) and *n*-Bu₃P (32 mg, 0.16 mmol) were added in a solution of 9 (84 mg, 0.13 mmol) in 1 ml of THF at room temperature. The solution was stirred at room temperature for 4 hours. 2-nitrophenyl selenocyanate (72 mg, 0.32 mmol) and *n*-Bu₃P (64 mg, 0.32 mmol) were then added at room temperature. The suspension was stirred at room temperature for 30 minutes. After that time, the solvent was removed at reduced pressure. Celite^{®} and AcOEt were added to the reaction crude and the solvent was removed at reduced pressure. Product 11 was isolated after column chromatography separation (hexane/AcOEt, 10:1), being obtained as a yellow oil.
¹H-NMR: δ 8.31 (1H, dm, *J* = 8.5 Hz); 7.47 (3H, m); 7.31 (5H, m); 5.93 (1H, s); 5.60 (1H, d, *J* = 5.2 Hz); 5.44 (1H, s, NH); 4.82 (1H, part A *syst. AB, J* = 3.0 Hz); 4.76 (1H, part *B syst. AB, J* = 3.0 Hz); 4.70 (2H, s); 4.52 (1H, part A *syst. AB, J* = 9.2 Hz); 3.63 (1H, part *B syst. AB, J* = 9.2 Hz); 3.48 (2H, s); 2.50 (1H, dd, *J* = 14.2, 5.2 Hz); 1.74 (1H, d, *J* = 14.2 Hz); 1.44 (6H, s); 1.08 (28H, m).
LRMS (*API-IS⁺*): *m*/*z* 821 (M+H)⁺, 843 (M+Na)⁺.

### Example 26

### Preparation of rac-(1R,2S,3S,4R,5R,8R)-2,4,5,8-tetrahydroxy-4,6-(hydroxymethyl)-6-methylidene-1-nitro-4,10-O-(propane-2,2-diyl)-2,8-O-(1,1,3,3-tetraisopropyldisiloxane-1,3-diyl)-9-oxabicyclo[3.3.1]nonane (12)

### Method A

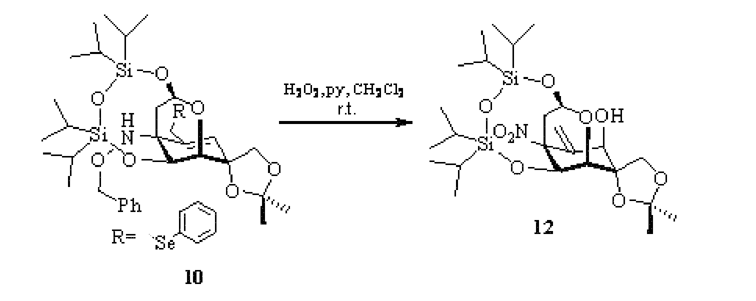

Pyridine (12 mg, 0.16 mmol) and H₂O₂ (18 mg, 0.54 mmol) were added in a solution of 10 (60 mg, 0.08 mmol) in 1.5 ml of CH₂Cl₂ at room temperature. The mixture was stirred at room temperature for 40 minutes. After that time, Na₂HPO₄ (0.1 M, 2 ml) was added at room temperature. The phases were separated in an extraction funnel. The aqueous phase was extracted with AcOEt (4 x 2 ml). The combined organic extracts were dried with anhydrous Na₂SO₄, they were filtered and the solvent was removed at reduced pressure. Celite^{®} and AcOEt were added to the reaction crude and the solvent was removed at reduced pressure. Product 12 was isolated after column chromatography separation (hexane/AcOEt, 7:1), being obtained as a colorless oil.

### Method B

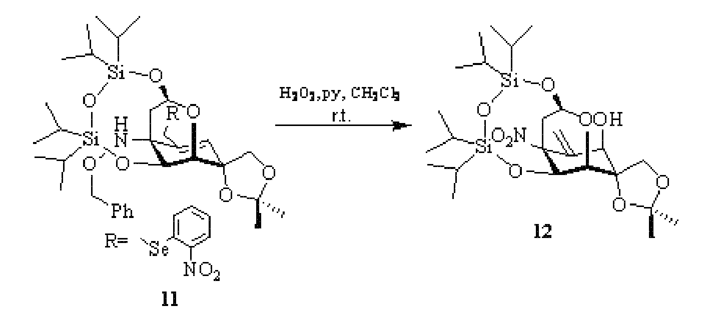

Pyridine (14 mg, 0.18 mmol) and H₂O₂ (21 mg, 0.63 mmol) were added in a solution of 11 (74 mg, 0.09 mmol) in 1.5 ml of CH₂Cl₂ at room temperature. The mixture was stirred at room temperature for 40 minutes. After that time, Na₂HPO₄ (0.1 M, 2 ml) was added at room temperature. The phases were separated in an extraction funnel. The aqueous phase was extracted with AcOEt (4 x 2 ml). The combined organic extracts were dried with anhydrous Na₂SO₄, they were filtered and the solvent was removed at reduced pressure. Celite^{®} and AcOEt were added to the reaction crude and the solvent was removed at reduced pressure. Product 12 was isolated after column chromatography separation (hexane/AcOEt, 7:1), being obtained as a colorless oil.
¹H-NMR: δ 5.85 (1H, s); 5.75 (1H, s); 5.53 (1H, dd, *J* = 5.2, 1.9 Hz); 5.36 (1H, d, *J* = 3.8 Hz); 4.99 (1H, d, *J* = 3.8 Hz); 4.24 (1H, s); 4.17 (1H, part A *syst. AB, J* = 9.6 Hz); 3.51 (1H, *part B syst. AB, J* = 9.6 Hz); 3.20 (1H, dd, *J* = 14.1, 5.2 Hz); 2.36 (1H, dd, *J* = 14.1, 1.9 Hz); 1.48 (3H, s); 1.42 (3H, s); 1.05 (28H, m).
LRMS (*API-IS⁺*) : *m*/*z* 513 (M-NO₂)⁺, 560 (M+H)⁺, 582 (M+Na)⁺ 1141 (2M+Na)⁺.

### Example 27

### Preparation of rac-(1R,2S,3S,4R, 8R*)-1-(benzyloxyamino)-2,4,8-trihydroxy-4 -(hydroxymethyl)-6-(phenylselanylmethyl)-4,10-O-(propane-2,2-diyl) -9-oxabicyclo[3.3.1]non-5-ene (13)

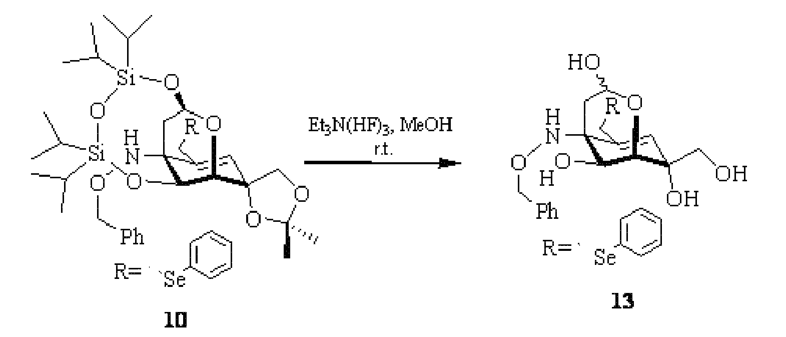

Et₃N(HF)₃ (404 mg, 2.51 mmol) was added in a suspension of 10 (54 mg, 0.07 mmol) in 4 ml of MeOH at room temperature. The mixture was stirred at room temperature for 16 hours. After that time, Et₃N(HF)₃ (404 mg, 2.51 mmol) was added at room temperature. The mixture was stirred at room temperature for 3 days. Na₂HPO₄ (0.1 M, 4 ml) was then added at room temperature. The phases were separated in an extraction funnel. The aqueous phase was extracted with AcOEt (5 x 4 ml). The combined organic extracts were dried with anhydrous Na₂SO₄, they were filtered and the solvent was removed at reduced pressure. Celite^{®} and AcOEt were added to the reaction crude and the solvent was removed at reduced pressure. After column chromatography separation (hexane/AcOEt, 1:3) a transparent oil was obtained. Et₃N(HF)₃ (224 mg, 1.39 mmol) was added in a solution of this oil (30 mg, 0.04 mmol) in 2 ml of MeOH at room temperature. The mixture was stirred at room temperature for 18 hours. After that time, the solvent was removed at reduced pressure. Celite^{®} and AcOEt were added to the reaction crude and the solvent was removed at reduced pressure. Product 13 was isolated after column chromatography separation (hexane/AcOEt, 1:3), being obtained as a white solid.
¹H-NMR: δ 7.51 (2H, m); 7.31 (5H, m); 7.21 (3H, m); 5.70 (1H, t, *J* = 1.2 Hz); 5.36 (1H, d, *J* = 3.3 Hz); 4.70 (2H, s); 4.55 (1H, S_{broad})4.54 (1H, d, *J* = 3.5 Hz); 4.11 (1H, d, *J* = 11.7 Hz); 3.59 (1H, dd, *J* = 3.5, 1.2 Hz); 3.06 (1H, dd, *J* = 11.7, 1.2 Hz); 2.47 (1H, d, *J* = 12.2 Hz); 1.89 (1H, dd, J = 12.2, 3.3 Hz).
LRMS (*API-IS⁺*) : *m*/*z* 476 (M-H₂O+H)⁺, 498 (M-H₂O+Na)⁺, 973 (2M-H₂O +Na)⁺.

## Claims

1. A compound of formula (II), stereoisomers, salts or solvates thereof, 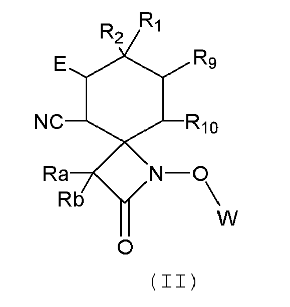 wherein
R₁ is selected from hydrogen, cyano, substituted or unsubstituted alkyl, substituted or unsubstituted alkenyl, substituted or unsubstituted alkynyl and NReRf, wherein Re and Rf are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxyl, substituted or unsubstituted aryloxyl, substituted or unsubstituted amino and halogen;
R₂ is selected from hydrogen, OH, or OPr; or
R₁ and R₂ together form a group selected from =O, alkylidene and -CH₂-O-Pr-O-;
E is selected from the group consisting of fluorine, chlorine, bromine, iodine, -SeR₁₃, -O-NR₁₃, -SR₁₄, PO (O-alkyl) ₂ and PO(O-aryl)₂, wherein
R₁₃ is selected from the group consisting of aryl and alkyl;
R₁₄ is selected from the group consisting of aryl, alkyl, -PO (O-alkyl)₂, -PO(O-aryl)₂, -C(=O)O-alkyl and -C(=O)O-aryl;
R₉ and R₁₀ are each independently selected from hydrogen, OH, OPr and =O; or together form an -O-Pr-O- group;
W is selected from the group consisting of -H, substituted or unsubstituted alkyl, substituted or unsubstituted aryl, substituted or unsubstituted arylalkyl, substituted or unsubstituted heterocyclyl and substituted or unsubstituted alkenyl;
Ra and Rb are each independently selected from H, OH, OPr, Se-aryl, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxyl, substituted or unsubstituted aryloxyl, substituted or unsubstituted amino and halogen; and
Pr is a hydroxyl protecting group.

2. A compound of formula (IIa), stereoisomers, salts or solvates thereof, 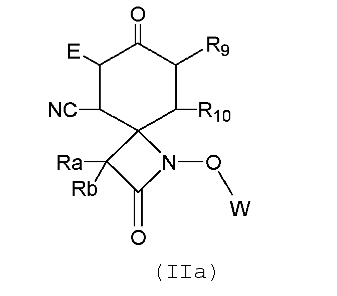 wherein
E, R₉, R₁₀, Ra, Rb and W are as have been defined in claim 1.

3. A compound of formula (III), stereoisomers, salts or solvates thereof, 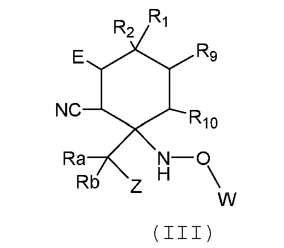 wherein
R₁, R₂, R₉, R₁₀, Ra, Rb, E and W are as defined in claim 1; and
Z is -COOH or -CHR₄R₅, wherein R₄ is hydrogen, and R₅ is OH or OPr; or R₄ and R₅ together form =O.

4. A compound of formula (IIIa), stereoisomers, salts or solvates thereof, 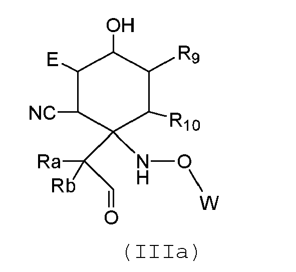 wherein
R₉, R₁₀, Ra, Rb, E and W are as defined in claim 1.

5. A compound of formula (IIIb), stereoisomers, salts or solvates thereof, 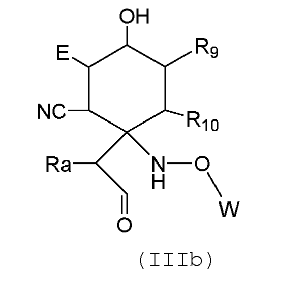 wherein
R₉, R₁₀, Ra, E and W are as defined in claim 1.

6. A compound of formula (IV), stereoisomers, salts or solvates thereof, 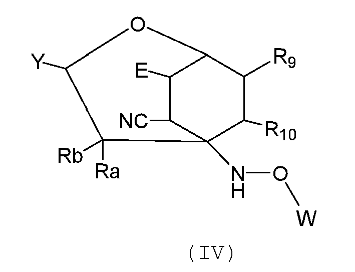 wherein
R₉, R₁₀, Ra, Rb, E and W are as defined in claim 1; and
Y is selected from the group consisting of -OR₆, -SR₆, Se-aryl, -N(R₆)₂, -+N (R₆)₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)-arylalkyl.

7. A compound of formula (V), stereoisomers, salts or solvates thereof, 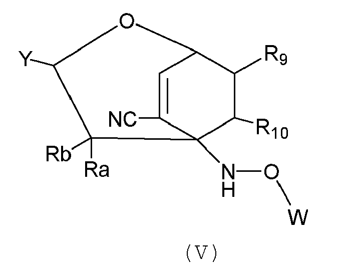 wherein
R₉, R₁₀, Ra, Rb and W are as defined in claim 1; and
Y is selected from the group consisting of -OR₆, -SR₆, Se-aryl, -N (R₆)₂, -+N(R₆)₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)- arylalkyl.

8. A compound of formula (VI), stereoisomers, salts or solvates thereof, 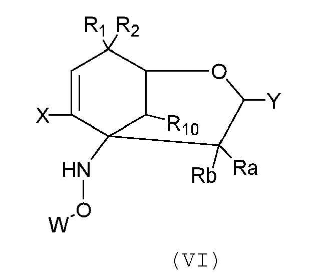 wherein
R₁, R₂, R₁₀, Ra, Rb and W are as defined in claim 1;
X is -C(=O) or -CN; and
Y is selected from the group consisting of -OR₆, -SR₆, Se-aryl, -N(R₆)₂, -+N(R₆)₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)- arylalkyl.

9. A compound of formula (VII), stereoisomers, salts or solvates thereof, 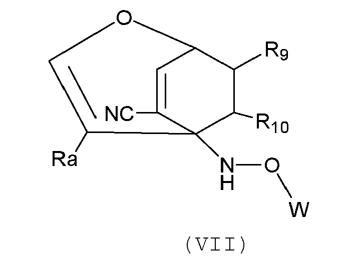 wherein
R₉, R₁₀, Ra and W are as defined in claim 1.

10. A compound of formula (VIII), stereoisomers, salts or solvates thereof, 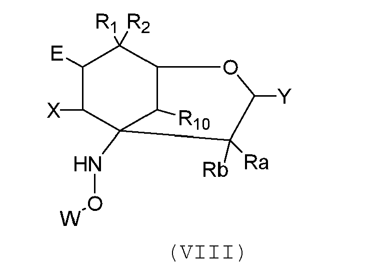 wherein
E, R₁, R₂, R₁₀, Ra, Rb and W are as defined in claim 1; X is -C(=O) or -CN; and
Y is selected from the group consisting of -OR₆, -SR₆, Se-aryl, -_{N}(R₆)₂, -+N(R₆)₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl,
- O-aryl, -O-arylalkyl, O-(C=O)-alkyl, O-(C=O)-aryl and O-(C=O)- arylalkyl.

11. A compound of formula (IX), stereoisomers, salts or solvates thereof, 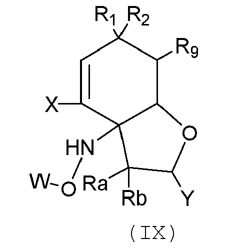 wherein
R₁, R₂, R₉, Ra, Rb and W are as defined in claim 1 X is -CH(=O) or -CN; and
Y is selected from the group consisting of -OR₆, -SR₆, Se-aryl, -N(R₆)₂, -+N(R₆)₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)- arylalkyl.

12. A compound of formula (Xa), stereoisomers, salts or solvates thereof 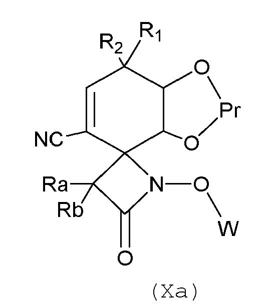 wherein
R₁, R₂, Ra, Rb, Pr and W are as defined in claim 1.

13. A compound of formula (XI), stereoisomers, salts or solvates thereof, 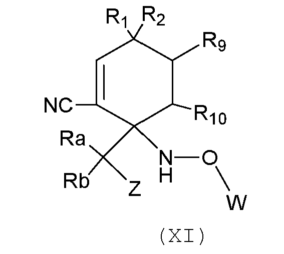 wherein
R₁, R₂, R₉, R₁₀, Ra, Rb, Z and W are as defined in claim 1.

14. A compound of formula (XII), stereoisomers, salts or solvates thereof, 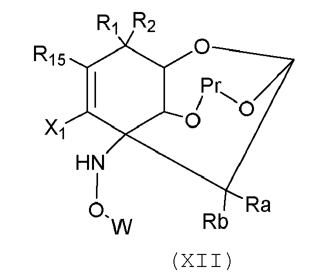 wherein
R₁, R₂, Pr, Ra, Rb and W are as defined in claim 1;
R₁₅ is -OH or -OPr, wherein Pr is as defined in claim 1; and
X₁ is selected from the group consisting of -CN, - COOR₁₆, -CON(R₁₆)₂, CHO, -CH₂OH, CH₂N(R₁₆)₂, -Se-Aryl, - Se(=O)-aryl, said aryl being able to be optionally substituted, -S-R₁₆, -S(=O)R₁₆, -CH(OR₁₆)₂, -CH (NHR₁₆) CN and - CH (OR₁₆) CN;
wherein R₁₆ is in each case independently selected from the group consisting of hydrogen, alkyl and aryl; two R₁₆ groups being able to form a linear or branched alkylidene group, optionally substituted with one or more carbonyl groups.

15. A compound of formula (XIII), stereoisomers, salts or solvates thereof, 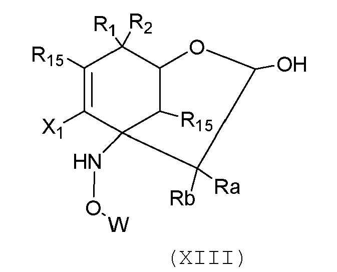 wherein
R₁, R₂, Ra, Rb and W are as defined in claim 1; X₁ is selected from the group consisting of -CN, -CHO, -CH₂OH, -Se-aryl, said aryl being able to be optionally substituted; and
each R₁₅ is independently -OH or -OPr, wherein Pr is as defined in claim 1.

16. A compound of formula (XIV), stereoisomers, salts or solvates thereof, 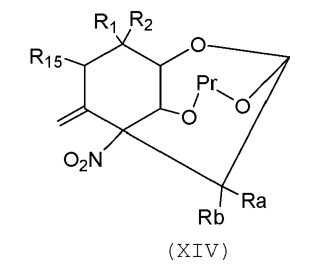 wherein
R₁, R₂, Ra, Rb and Pr are as defined in claim 1; and R₁₅ is -OH or -OPr.

17. The compound according to any of claims 1 to 16, wherein W is arylalkyl.

18. The compound according to claim 17, wherein W is CRcRd-aryl, wherein Rc and Rd are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxyl, substituted or unsubstituted aryloxyl, substituted or unsubstituted amino and halogen.

19. The compound according to claim 18, wherein Rc is other than hydrogen and Rd is hydrogen.

20. The compound according to claim 18, wherein Rc and Rd are both hydrogen.

21. The compound according to any of claims 1 to 20, wherein Ra is hydrogen and Rb is selected from the group consisting of halogen, preferably bromine, OH, OPr and Se-aryl.

22. The compound according to any of claims 1 to 20, wherein Ra and Rb are both hydrogen.

23. The compound according to any of claims 1 to 22, wherein Y is selected from OH, alkoxyl, ammonium and -NH(O-(C=O)-aryl).

24. The compound according to any of claims 1 to 23, wherein E is bromine or iodine, preferably bromine.

25. The compound according to any of claims 1 to 24, wherein R₉ and R₁₀ together form a group of formula -O-Si(R₁₁R₁₂)-O-Si(R₁₁R₁₂)-O-, wherein R₁₁ and R₁₂ are independently selected from the group consisting of hydrogen, substituted or unsubstituted alkyl, substituted or unsubstituted cycloalkyl, substituted or unsubstituted alkenyl, substituted or unsubstituted alkynyl , substituted or unsubstituted aryl and substituted or unsubstituted heterocyclyl.

26. The compound according to claim 25, wherein R₁₁ and R₁₂ are isopropyl.

27. The compound according to any of claims 1 to 26, wherein R₁ is alkyl-OPr.

28. The compound according to any of claims 1 to 27, wherein R₁ and R₂ together form =O, alkylidene or -CH₂-O-Pr-O-.

29. The compound according to claim 28, wherein Pr is - [(R₁₁)C(R₁₂)]-, wherein R₁₁ and R₁₂ are as have been defined in claim 25, preferably methyl.

30. The compound according to any of claims 1 to 29, wherein R₁ is hydrogen and R₂ is OH or OPr.

31. A method for synthesizing a compound of formula (II), stereoisomers, salts or solvates thereof as has been defined in claim 1 comprising a sequence A which comprises (ai) reacting a compound of formula (I) in the presence of a cyanide ion source, and (aii) reacting the intermediate obtained in the presence of an electrophile, or a sequence B which comprises (bi) reacting a compound of formula (I) in the presence of electrophile, and (bii) reacting the intermediate obtained in the presence of a cyanide ion source 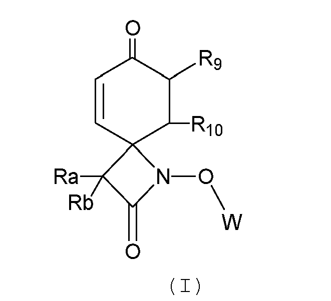 wherein R₉, R₁₀, Ra, Rb and W are as have been defined in claim 1.

32. The method according to claim 31, additionally comprising one or more steps which are selected from the group consisting of:
a) reducing the ketone to hydroxyl group, optionally followed by protecting and/or inverting the configuration of said hydroxyl group;
b) olefinating the ketone to obtain an alkylidene group, optionally followed by dihydroxylation;
c) olefinating the ketone to obtain an alkylidene group, optionally followed by dihydroxylation, and subsequent protection of at least one of the hydroxyl groups formed;
d) protecting the ketone;
e) alkylating the ketone; and
f) aminating the ketone.

33. A method for synthesizing a compound of formula (III), stereoisomers, salts or solvates thereof as defined in claim 3, which comprises reacting a compound of formula (II) in the presence of a hydride.

34. The method according to claim 33, comprising oxidizing the aldehyde group of position 10 according to the TTX numbering to acid group.

35. A method for synthesizing a compound of formula (IV), stereoisomers, salts or solvates thereof as defined in claim 6, comprising reacting a compound of formula (IIIa) in the presence of a base.

36. A method for synthesizing a compound of formula (IV), stereoisomers, salts or solvates thereof as defined in claim 6, Ra being a -Se-aryl group, comprising reacting a compound of formula (IIIb) in the presence of halogen-Se-aryl or aryl-Se-Se-aryl, and of a base, preferably a cyclic amine, preferably morpholine.

37. A method for synthesizing a compound of formula (V), stereoisomers, salts or solvates thereof as defined in claim 7, comprising reacting a compound of formula (IIIa) in the presence of a base.

38. A method for synthesizing a compound of formula (V), stereoisomers, salts or solvates thereof as defined in claim 7, Rb being a -Se-aryl group, comprising reacting a compound of formula (IIIb) in the presence of halogen-Se-aryl or aryl-Se-Se-aryl, and a base, preferably a cyclic amine, preferably morpholine.

39. The method according to claim 35, 36, 37 or 38, wherein the compound of formula (V) or of formula (IV) obtained is reacted in acid medium in the presence of a reagent which is selected from the group consisting of HOR₆, HSR₆, Se-aryl, aryl-Se-Se-aryl, HN (R₆) ₂, N (R₆) ₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and
R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)- arylalkyl.

40. A method for obtaining a compound of formula (VI), stereoisomers, salts or solvates thereof as defined in claim 8, which comprises reacting a compound of formula (V) in acid medium in the presence of a reagent which is selected from the group consisting of HOR₆, HSR₆, Se-aryl, aryl-Se-Se-aryl, HN (R₆) ₂, N (R₆) ₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and
R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)- arylalkyl.

41. A method for obtaining a compound of formula (VII), stereoisomers, salts or solvates thereof as defined in claim 9, which comprises reacting a compound of formula (V) with a base.

42. A method for obtaining a compound of formula (VIII), stereoisomers, salts or solvates thereof as defined in claim 10, which comprises reacting a compound of formula (IV) in acid medium in the presence of a reagent which is selected from the group consisting of HOR₆, HSR₆, Se-aryl, aryl-Se-Se-aryl, HN (R₆) ₂, N (R₆) ₃ and -NHR₇,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl; and
R₇ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, -O-alkyl, -O-aryl, -O-arylalkyl, O-(C=O)-alkyl, O-(C=O)-aryl and O-(C=O)- arylalkyl.

43. A method for obtaining a compound of formula (XI), stereoisomers, salts or solvates thereof as defined in claim 13, which comprises reacting a compound of formula (X), stereoisomers, salts or solvates thereof in the presence of a hydride, preferably with DIBAL, 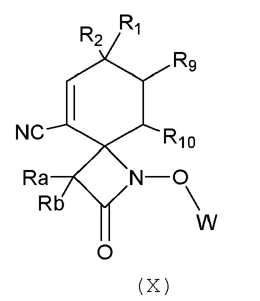 wherein R₁, R₂, R₉, R₁₀, Ra, Rb and W are as defined in claim 1.

44. A method for obtaining a compound of formula (XII), stereoisomers, salts or solvates thereof as defined in claim 14, which comprises reacting a compound of formula (XI) in the presence of a base.

45. The method according to any of claims 28 to 32 or according to claim 44, wherein the base is selected from the group consisting of phosphazenes, DBU, DABCO, cyclic aliphatic amines and trialkylamines.

46. The method according to reaction 45, wherein the base is DBU.

47. A method for obtaining a compound of formula (XIII), stereoisomers, salts or solvates thereof as defined in claim 15, which comprises deprotecting the hydroxyls of positions 8 and 10 according to the TTX numbering, removing the Pr group of a compound of formula (XII), stereoisomers, salts or solvates thereof as has been defined in claim 14.

48. The method according to claim 47, wherein R₉ and R₁₀ together form a group of formula -O-Si (R₁₁R₁₂) -O-Si (R₁₁R₁₂) -O-, wherein R₁₁ and R₁₂ are independently selected from the group consisting of hydrogen, substituted or unsubstituted alkyl, substituted or unsubstituted cycloalkyl, substituted or unsubstituted alkenyl, substituted or unsubstituted alkynyl , substituted or unsubstituted aryl and substituted or unsubstituted heterocyclyl, preferably wherein R₁₁ and R₁₂ are isopropyl.

49. A method for obtaining a compound of formula (XIV), stereoisomers, salts or solvates thereof as defined in claim 16, which comprises reacting a compound of formula (XII), stereoisomers, salts or solvates thereof as has been defined in claim 14, wherein X₁ is -Se-aryl or -SR₁₆, wherein R₁₆ is as has been defined in claim 14, in the presence of a peroxide, preferably in the presence of peroxide hydroxide.

50. The method according to any of claims 31 to 49, comprising the simultaneous deprotection of R₉ and R₁₀.

51. The method according to any of claims 31 to 50, which comprises introducing a -Se-aryl group in position 9 according to the TTX numbering, and optionally its subsequent reaction with a group of formula OR₈, wherein R₈ is selected from the group consisting of - (C=O) R₆ and -NHR₆,
wherein R₆ is selected from the group consisting of substituted or unsubstituted alkyl, substituted or unsubstituted cycloalkyl, substituted or unsubstituted alkenyl, substituted or unsubstituted aryl, substituted or unsubstituted arylalkyl and substituted or unsubstituted heterocyclyl.

52. Use of a compound of formula (II), (IIa), (III), (IIIa), (IIIb), (IV), (V), (VI), (VII), (VIII), (IX), (Xa), (XI), (XII), (XIII) or (XIV) as intermediate in the synthesis of TTX and analogs thereof.

53. A method for synthesizing TTX which comprises obtaining a compound of formula (XIII), stereoisomers, salts or solvates thereof as defined in claim 15, comprising deprotecting the hydroxyls of positions 8 and 10 according to the TTX numbering, removing the Pr group of a compound of formula (XII), stereoisomers, salts or solvates thereof as has been defined in claim 14.

54. A method for synthesizing TTX which comprises obtaining a compound of formula (XIV), stereoisomers, salts or solvates thereof as defined in claim 16, comprising reacting a compound of formula (XII), stereoisomers, salts or solvates thereof as has been defined in claim 14, wherein X₁ is -Se-aryl or -SR₁₆, wherein R₁₆ is as has been defined in claim 14, in the presence of a peroxide, preferably in the presence of peroxide hydroxide.

55. The method according to claim 53 or 54, wherein said compound of formula (XII), stereoisomers, salts or solvates thereof as defined in claim 14, comprises reacting a compound of formula (XI) in the presence of a base.

56. The method according to claim 55, wherein obtaining said compound of formula (XI), stereoisomers, salts or solvates thereof as defined in claim 13, comprises reacting a compound of formula (X), stereoisomers, salts or solvates thereof as defined in claim 43 in the presence of a hydride.
